(19) **Europäisches Patentamt**

**European Patent Office**

**Office européen des brevets**

(11) **EP 0 619 752 B2**

(12) ## NEW EUROPEAN PATENT SPECIFICATION

(45) Date of publication and mention
of the opposition decision:
**18.10.2000 Bulletin 2000/42**

(45) Mention of the grant of the patent:
**14.05.1997 Bulletin 1997/20**

(21) Application number: **93912085.3**

(22) Date of filing: **23.03.1993**

(51) Int. Cl.⁷: $B01D\ 21/26$, $B01D\ 33/00$,
$A61M\ 1/36$

(86) International application number:
**PCT/US93/02854**

(87) International publication number:
**WO 94/08688 (28.04.1994 Gazette 1994/10)**

(54) **COMPACT ENHANCED YIELD BLOOD PROCESSING SYSTEMS**

KOMPAKTE BLUTVERARBEITUNGSSYSTEME MIT VERBESSERTER AUSBEUTE

SYSTEMES COMPACTS DE TRAITEMENT DU SANG A RENDEMENT AMELIORE

(84) Designated Contracting States:
**BE DE FR GB IT**

(30) Priority: **22.10.1992 US 964771**

(43) Date of publication of application:
**19.10.1994 Bulletin 1994/42**

(73) Proprietor:
**BAXTER INTERNATIONAL INC.**
**Deerfield, IL 60015 (US)**

(72) Inventor: **BROWN, Richard, I.**
**Northbrook, IL 60062 (US)**

(74) Representative:
**MacGregor, Gordon**
**Eric Potter Clarkson,**
**Park View House,**
**58 The Ropewalk**
**Nottingham NG1 5DD (GB)**

(56) References cited:
**WO-A-89/00084**          **US-A- 4 146 172**
**US-A- 4 834 890**

EP 0 619 752 B2

**Description**

<u>Field of the Invention</u>

The invention relates to centrifugal processing systems and apparatus.

<u>Background of the Invention</u>

Today blood collection organizations routinely separate whole blood by centrifugation into its various therapeutic components, such as red blood cells, platelets, and plasma.

Conventional blood processing systems and methods use durable centrifuge equipment in association with single use, sterile processing chambers, typically made of plastic. The centrifuge equipment introduces whole blood into these chambers while rotating them to create a centrifugal field.

Whole blood separates within the rotating chamber under the influence of the centrifugal field into higher density red blood cells and platelet-rich plasma. An intermediate layer of white blood cells and lymphocytes forms an interface between the red blood cells and platelet-rich plasma.

Conventional blood processing methods use durable centrifuge equipment in association with single use, sterile processing systems, typically made of plastic. The operator loads the disposable systems upon the centrifuge before processing and removes them afterwards.

Conventional centrifuges often do not permit easy access to the areas where the disposable systems reside during use. As a result, loading and unloading operations can be time consuming and tedious.

Disposable systems are often preformed into desired shapes to simplify the loading and unloading process. However, this approach is often counterproductive, as it increases the cost of the disposables.

<u>Summary of the Invention</u>

The invention provides improved blood processing chambers as in Claims 1 and 2, which create unique dynamic flow conditions and are compact and easily handled.

One aspect of the invention provides a chamber for use in a rotating field to separate blood components. The chamber includes a separation channel having a low-G side wall radially spaced from the rotational axis, a high-G side wall radially spaced apart circumferentially about the rotation axis. An inlet port near the first end wall introduces blood into the channel for flow circumferentially about the rotational axis from the first end wall toward the second end wall for separation into two blood components.

In this aspect of the invention, the outlet ports are juxtaposed next to the inlet port near the first end wall for conveying the separated blood components from the channel. In this way, the fluid flow tubing associated with the chamber is located within a single compact region of the chamber. This simplifies handling of the chamber, particularly when loading and unloading the chamber in a processing centrifuge.

The compact chamber that embodies the features of the invention directs one separated blood component to a collection region near the second end wall. An enclosed interior collection passage within the channel that leads from the collection region and directs the one collected component to the outlet port for transport from the chamber.

The chamber includes a barrier surface near the second end wall for creating a restricted inlet between the collection region and the collection passage.

Another aspect of the invention provides a chamber defining a separation zone that is divided into contiguous first and second separation channels.

In the arrangement, each channel includes an inlet port near its associated first end wall for introducing blood into the channel for flow circumferentially about the rotational axis from the first end wall toward the second end wall for separation. Each channel also includes at least one outlet port juxtaposed its associated inlet port for conveying a separated blood constituent from the associated channel.

Thus, according to the invention, the inlet and outlet ports of the two separation channels are mutually juxtaposed in a compact region on the chamber.

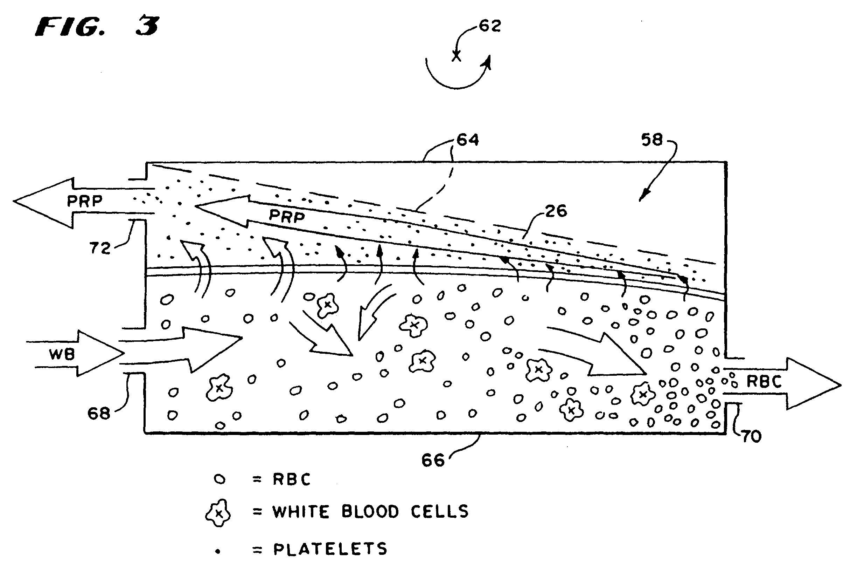

Hematocrit indicates the volume of red blood cells per unit volume of whole blood. A typical healthy donor has a predonation hematocrit of about 42.5%. The hematocrit of the blood lying on the boundary between the red blood cells and plasma along the interface is called "surface hemotocrit" (see fig.3).

<u>Brief Description of the Drawings</u>

Fig. 1 is a diagrammatic view of an enhanced yield circumferential flow processing chamber that embodies the features of the invention;

Fig. 2 is a diagrammatic view of the chamber shown in Fig. 11 operating in a centrifugation field;

Fig. 3 is a diagrammatic view of the interior of the chamber shown in Fig. 11 when processing whole blood within the centrifugation field;

Figs. 4 and 5 are diagrammatic views of prior art circumferential flow blood processing chambers;

Fig. 6 is a plan view of a blood processing assembly that incorporates an enhanced yield circumferential flow processing chamber that embodies the features of the invention;

Fig. 7 is a view of the interior of the blood processing assembly shown in Fig. 6, taken between the low-G and high-G walls radially along the centrifugation field;

Fig. 8 is a plan view of an alternative blood processing assembly that incorporates an enhanced yield circumferential flow processing chamber that embodies the features of the invention;

Fig. 9 is a view of the interior of the blood processing assembly shown in Fig. 8, taken between the low-G and high-G walls radially along the centrifugation field;

Fig. 10 is a side view of a centrifuge that can be used in association with either one of the blood processing assemblies shown in Figs. 6/7 or 8/9, showing the bowl and spool assemblies in their upraised and separated position;

Fig. 11 is a side view of the centrifuge shown in Fig. 10, showing the bowl and spool assemblies in their suspended and operating position;

Fig. 12 is an enlarged perspective view of one of the blood processing assemblies shown in Figs. 6/7 or 8/9 being wrapped for use about the spool of the centrifuge shown in Fig. 10;

Fig. 13 is an enlarged perspective view, with portions broken away, of one of the blood processing assemblies shown in Figs. 6/7 or 8/9 mounted for use on the bowl and spool assemblies of the centrifuge shown in Fig. 10;

Fig. 14 is a top interior section view, taken generally along line 14-14 in Fig. 13, of the processing chamber formed by the bowl and spool assemblies of the centrifuge shown in Fig. 10;

Figs. 15A/B/C are enlarged perspective views of an interior ramp used in association with either one of the blood processing assemblies shown in Figs. 6/7 or 8/9 for controlling flow of PRP from the chosen assembly;

Fig. 16 is a view of the vortex conditions generated within the blood processing assembly shown in Figs. 6/7 during use;

Fig. 17 is a single needle platelet collection system that can be used in association with either one of the blood processing assemblies shown in Figs. 6/7 or 8/9;

Fig. 18 is a double needle platelet collection system that can be used in association with either one of the blood processing assemblies shown in Figs. 6/7 or 8/9;

Fig. 19 is a plasma recirculation control system that can be used in association with either one of the blood processing systems shown in Figs. 17 or 18;

Fig. 20 is a perspective view, with portions broken away and in section, of an interface control system mounted on the rotating (one omega) portion of the centrifuge shown in Figs. 10 and 11 and used in association with the ramp shown in Fig. 15;

Fig. 21A is an enlarged perspective view of the rotating interface viewing head associated with the interface control system shown in Fig. 20;

Fig. 21B is a side section view showing the interior of rotating interface viewing head shown in Fig. 21A;

Fig. 22 is a schematic view of the light intensity control circuit associated with the interface control system shown in Fig. 20;

Figs. 23A/B/C are a series of diagrammatic views showing the operation of the interface control system shown in Fig. 20 during rotation of the centrifuge assembly;

Figs. 24A/B are flow charts showing the operation of the interface control circuit associated with the interface control system shown in Fig. 20;

Figs. 25A/B show, respectively, the platelet counts and mean platelet volumes sampled during a 45 minute procedure using a separation chamber that embodies the features of the invention; and

Figs. 26A/B show, respectively, the platelet counts and mean platelet volumes sampled during a 45 minute procedure using another separation chamber than embodies the features of the invention.

## I. ENHANCED YIELD CIRCUMFERENTIAL FLOW CRAMBERS

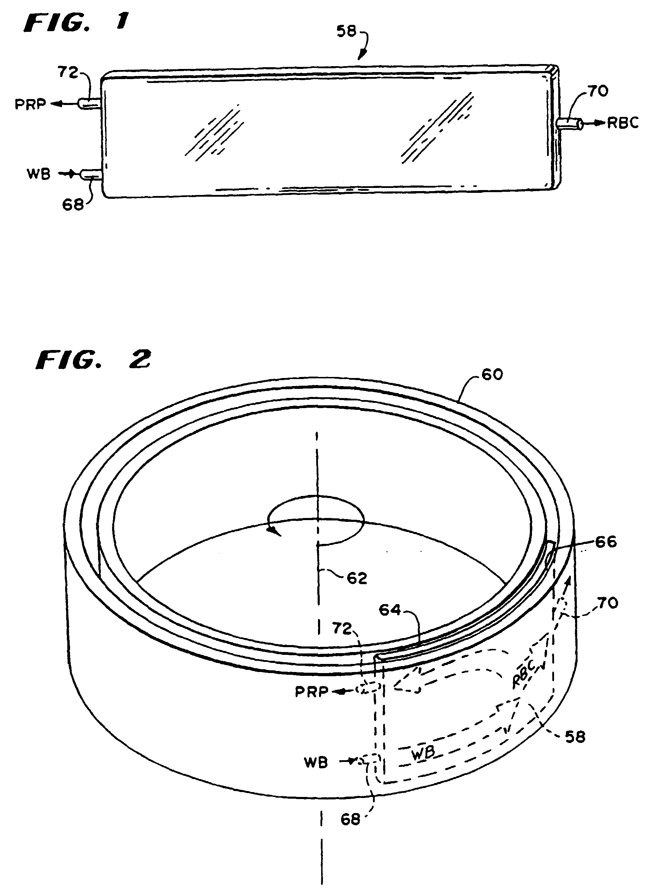

Figs. 1 to 3 show, in diagrammatic fashion, a circumferential flow centrifugal blood processing chamber 58 that embodies the features of the invention.

In use, the chamber 58 rotates on a rotor 60 about an axis 62 (see Fig. 2), to thereby create a centrifugal field within the chamber 58. The centrifugal field extends radially from the axis through the chamber 58. As Fig. 3 shows, the chamber wall 64 closest to the axis constitutes the low-G wall, and the chamber wall 66 farthest from the axis constitutes the high-G wall.

While rotating, the chamber 58 receives WB through a first port 68. The WB follows a circumferential flow path in the chamber 58; that is, it flows in a circumferential path about the rotational axis 62 (as Fig. 2 best shows). For this reason, the chamber 58 is called a circumferential flow blood processing chamber.

In this geometry, the transverse top and bottom edges of the chamber 58 (which lie along the circumferential flow path) are usually longer than the longitudinal side edges (which lie across the circumferential flow path). The circumferential flow chamber 58 usually forms the shape of a tube that is elongated in the direction of rotation. Still, other configurations defining a circumferential flow path can be used.

WB separates within the tubular chamber 58 under the influence of the centrifugal field into RBC and PRP. As Fig. 3 shows, the higher density RBC move toward the high-G wall 66, displacing the lighter density PRP toward the low-G wall 64. An interface 26 forms between them. A second port 70 draws the RBC from the chamber 58 for collection. A third port 72 draws the PRP from the chamber 58 for collection.

According to the invention, the PRP collection port 72 and the WB inlet port 68 are juxtaposed so that the PRP exits the circumferential flow chamber 58 in the same region where WB enters the chamber 58. In the illustrated embodiment, as shown in Fig. 1, the PRP collection port 72 is located along the same longitudinal side edge of the circumferential flow chamber 58 as the WB inlet port 68.

Also according to the invention, the RBC collection port 70 and the PRP collection port 72 are arranged so that PRP exits the chamber 58 in a region opposite to the region where RBC exit the chamber 58, relative to the circumferential flow of WB in the chamber 58. In the illustrated embodiment, as Fig. 1 shows, the RBC collection port 70 is located on the longitudinal side edge that is opposite to longitudinal side edge where the WB inlet and PRP collection ports are located.

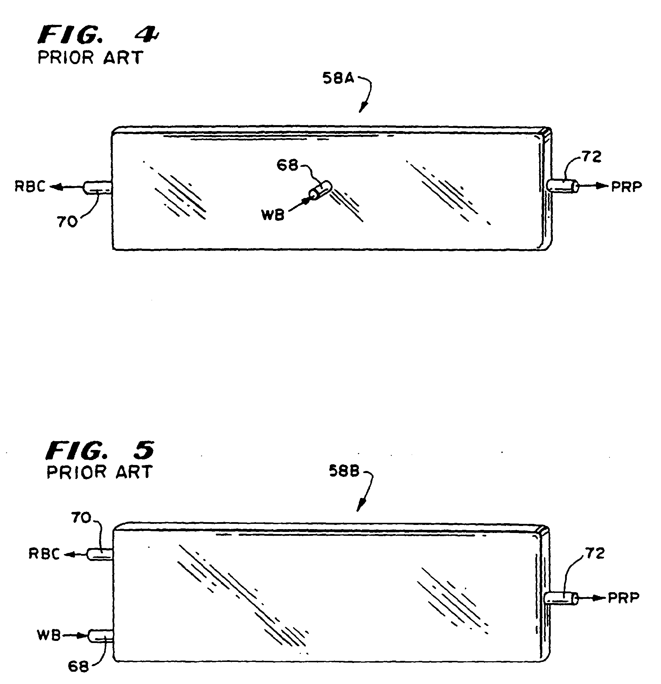

The chamber 58 shown in Figs. 1 to 3 differs significantly from prior circumferential flow blood separation chambers 58A and 58B, which are shown in Figs. 4 and 5. The prior circumferential flow chambers 58A/B purposely located the PRP collection port 72 away from the W/B inlet port 68.

In the prior circumferential flow chamber 58A shown in Fig. 4, the PRP collection port 72 occupies one side edge, diametrically opposite to the RBC collection port 70, which occupies the other side edge. In this construction, the WB inlet port 68 is located in a side wall of the chamber 58A between the two side edges.

In the prior circumferential flow chamber 58B shown in Fig. 5, the PRP collection port 72 occupies one side edge, while the WB inlet port 68 and the RBC outlet port occupies the opposite side edge, oppositely spaced away from the PRP collection port 7 relative to the circumferential flow of WB in the chamber 58B.

In both the Fig. 4 construction and the Fig. 5 construction, no ports are located on the top and bottom transverse edges of the chamber 58B. Neither chamber 58A and 58B has a port with an axis that extends parallel to the axis of rotation.

Fig. 3 diagrammatically shows the enhanced platelet separation effect due to the adjacent positions of the WB inlet port 68 and the PRP collection port 72 in the circumferential flow chamber 58 that embodies the invention.

As Fig. 3 shows, the PRP collection port 72 draws PRP from the chamber 58 where velocity at which the RBC settle toward the high-G wall 66 in response to centrifugal force is the greatest, i.e., next to the WB inlet port 68. Here, too, is where the radial plasma velocity is the greatest to lift platelets from the interface 26, and to keep them in suspension within the plasma for transport out the PRP collection port 72.

The WB inlet port 68 is oppositely spaced from the RBC collection port 70 (in the circumferential flow direction), forcing the RBC to traverse the entire axial length of the chamber 58, thereby maximizing their exposure to the centrifugal separation forces. The isolation between the RBC collection port 70 and the PRP collection port 72 also directs the RBC toward the RBC collection port 70, while directing the PRP stream in the opposite direction toward the PRP collection port 72.

The low-G wall 64 is preferably displaced inward toward the interface 26 near the RBC collection port 70. As a result, the radial distance between the low-G wall 64 and interface 26 is greater near the PRP collection port 72 than near the RBC collection port 70.

The displaced low-G wall 64 causes the lighter plasma to move along the interface 26 swiftly away from the relatively more confined region next to the RBC collection port 70, toward the relatively more open region next to the PRP collection port 72. The beneficial effect results that the circumferential plasma flow drags the interface 26 -- and larger, faster settling platelets entrapped within it -- continuously toward the PRP collection port 72, where the radial plasma velocities are the greatest to supply the greatest elution effect. The counterflow patterns also serve to circulate the other heavier components of the interface (lymphocytes, monocytes, and granulocytes) back into the RBC mass, away from the PRP stream.

As Fig. 3 shows, the low-G wall 64 continuously tapers in the direction of the circumferential flow path, e.g., away from the PRP collection port 72 and in the direction of axial flow path of the WB. The same result can be obtained without continuously or uniformly tapering the low-G wall 64 along the entire length of the axial flow path between the PRP collection port 72 and the RBC collection port 70. The low-G wall 64 can begin its taper farther away from the PRP collection port 24 than Fig. 3 shows, closer to the region of the RBC collection port 70.

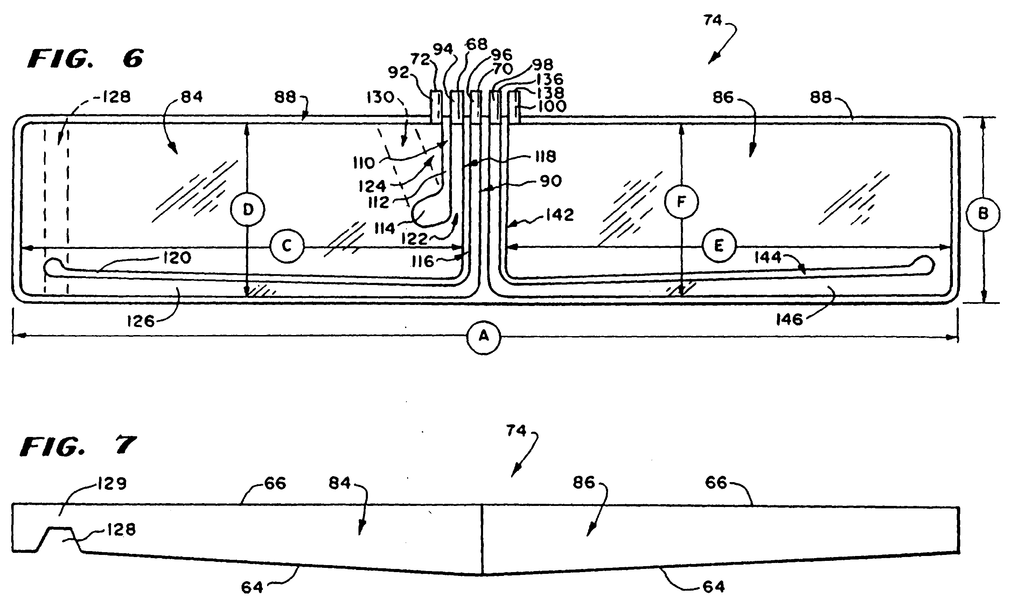

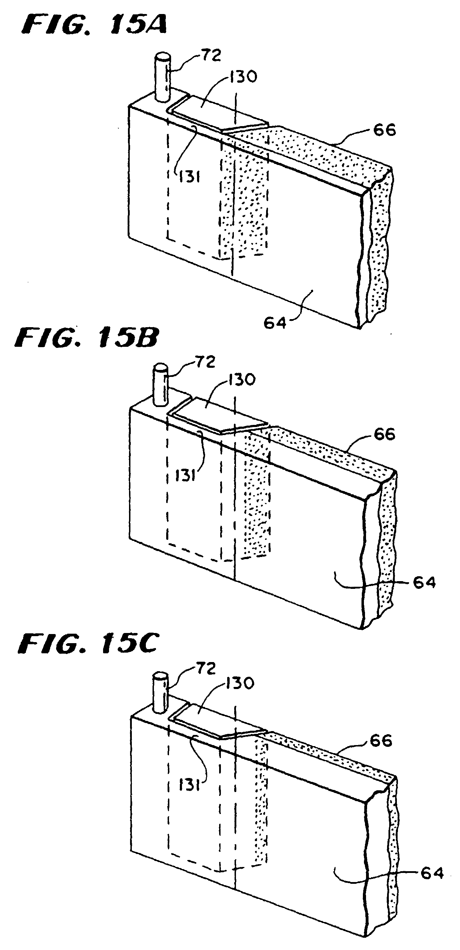

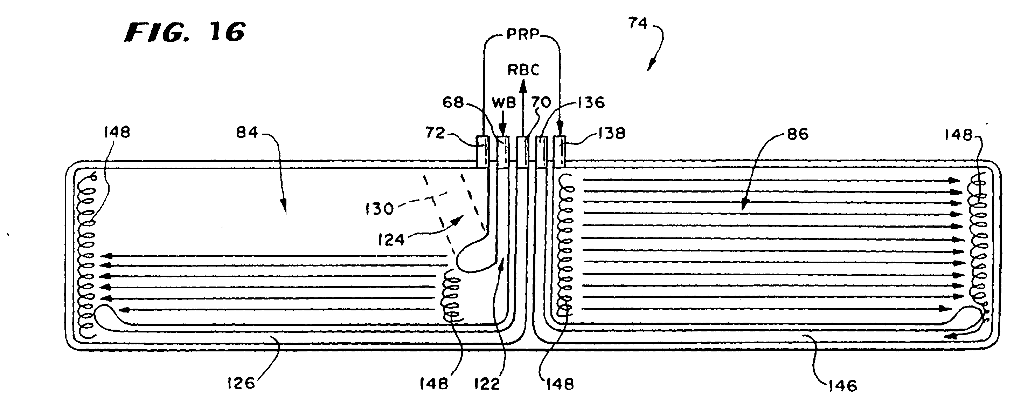

The circumferential flow chamber 58 that embodies the invention can be variously constructed. Figs. 6 and 7 show the physical construction of one preferred circumferential flow chamber assembly 74 that embodies the features of the invention. Figs. 15 and 16 show the physical construction of an alternative circumferential flow assembly 76.

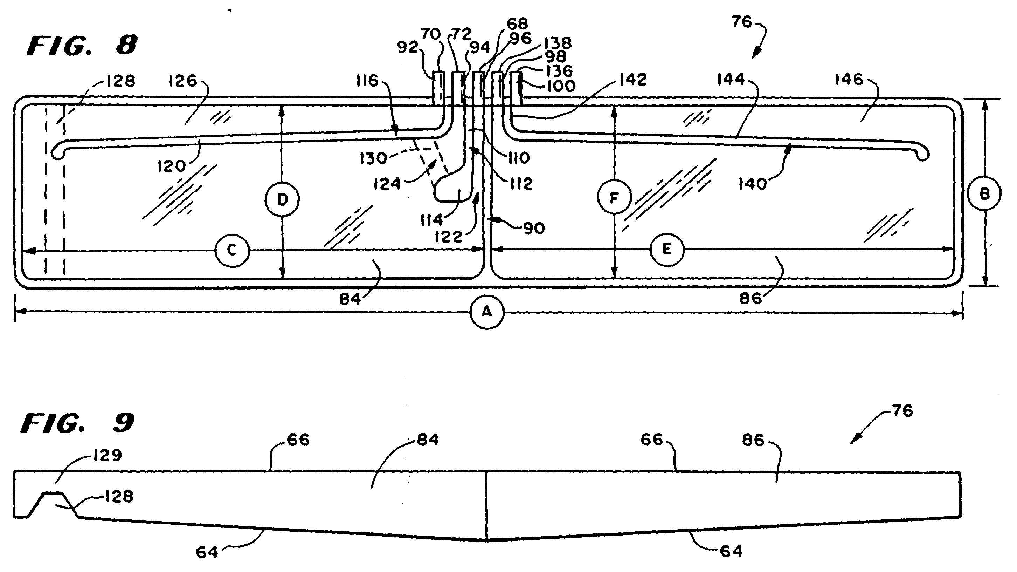

Either assembly 74 or 76 can be used in association with a blood processing centrifuge 78, like tat shown in Figs. 8 and 9. Further details of this centrifuge construction are set forth in EP-A-0572656.

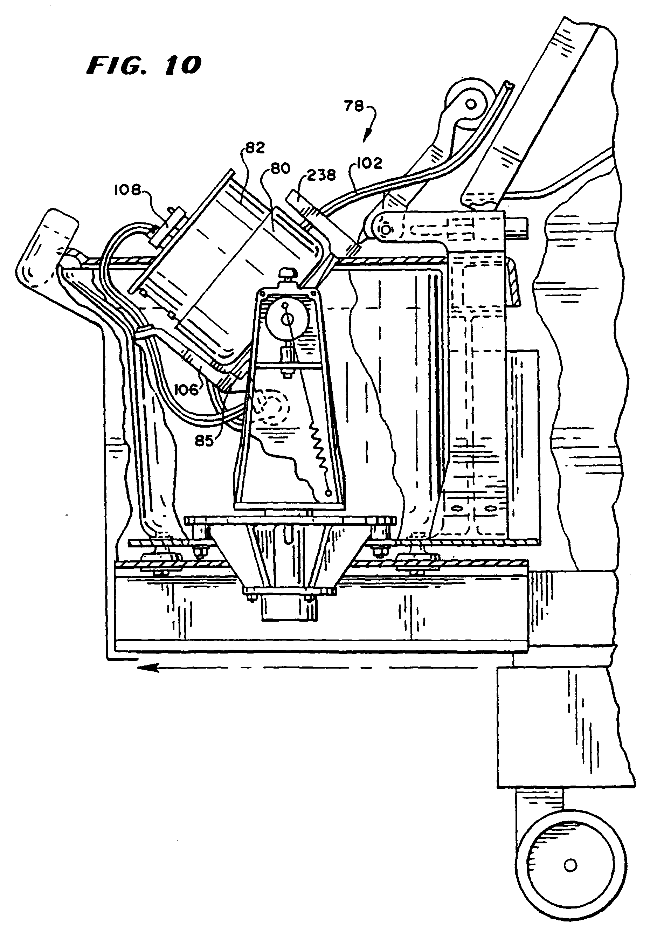

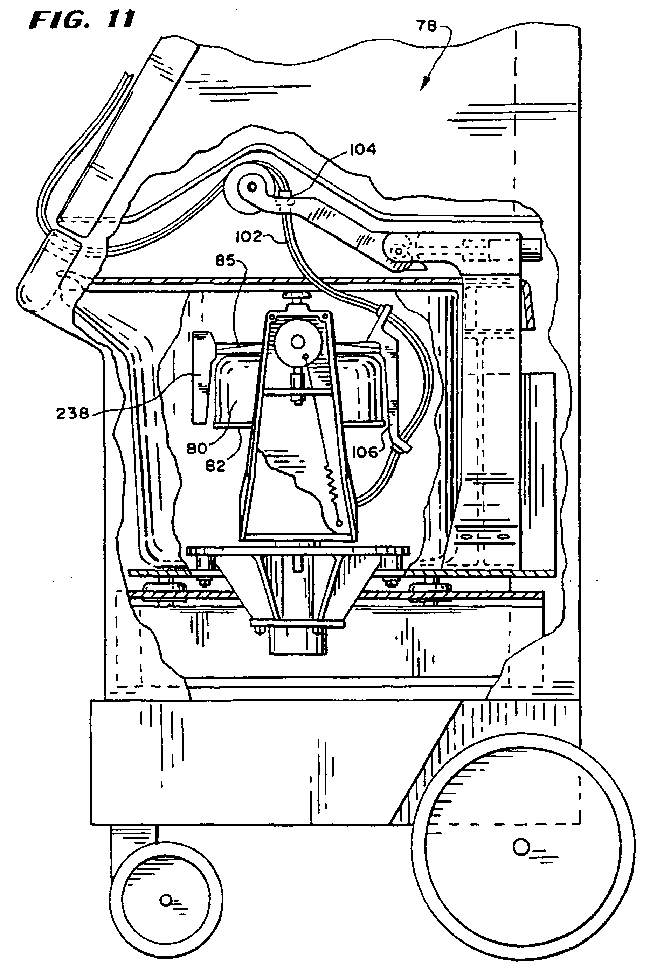

As Fig. 10 shows, the centrifuge 78 includes a bowl element 80 and a spool element 82. The bowl and spool elements 80 and 82 can be pivoted on a yoke 85 between an upright position, as Fig. 10 shows, and a suspended position, as Fig. 11 shows.

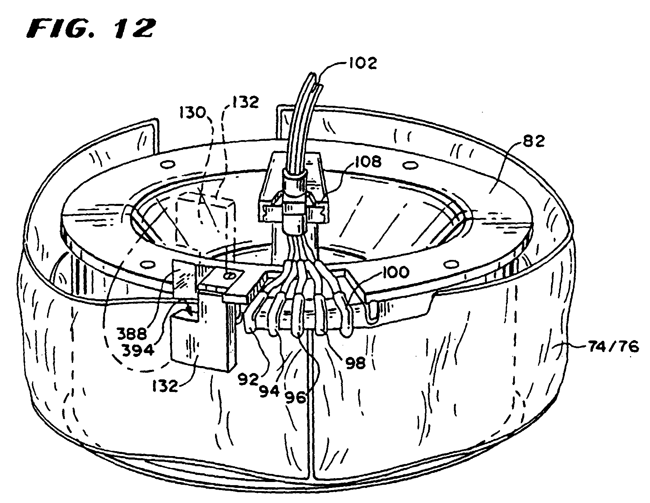

When upright, the bowl and spool elements 80 and 82 are presented for access by the user. A mechanism permits the spool and bowl elements 80 and 82 to assume a mutually separated position, as Fig. 10 shows. In this position, the spool element 80 is at least partially out of the interior area of the bowl element 82 to expose the exterior spool surface for access. As Fig. 12 shows, when exposed, the user can wrap either circumferential flow chamber assembly 74 or 76 about the spool element 82.

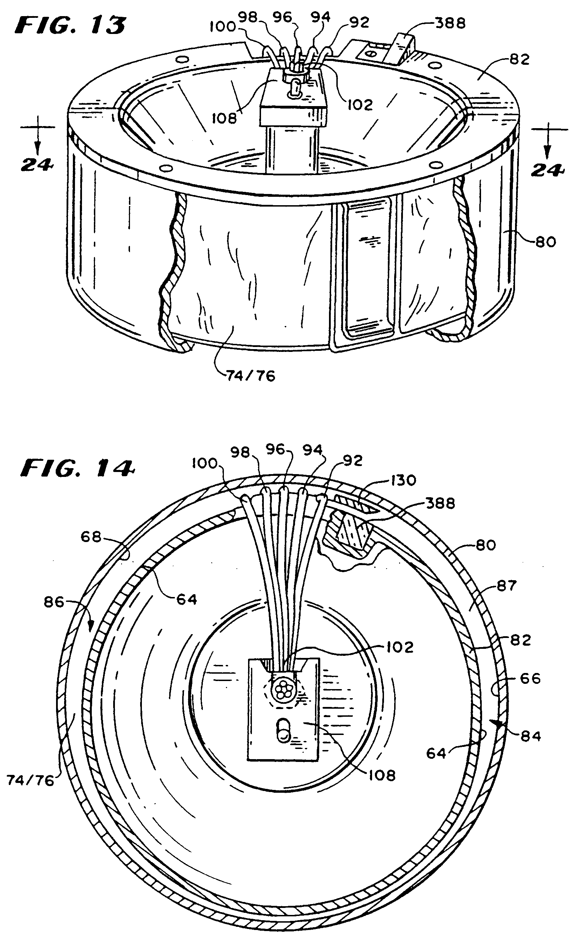

The mechanism also permits the spool and bowl elements 80 and 82 to assume a mutually cooperating position, as Fig. 13 shows. In this position, the spool element 82 and the chosen circumferential flow chamber assembly 74 or 76 are enclosed within the interior area of the bowl element 80, as Fig. 13 shows. A processing chamber 83 is formed between the interior of the bowl element 80 and the exterior of the spool element 82. The chosen circumferential flow chamber assembly 74 or 76 is carried with and assumes the contours of the processing chamber.

When closed, the spool and bowl elements 80 and 82 can be pivoted as an assembly into a suspended position, as Fig. 11 shows. When suspended, the bowl and spool elements 80 and 82 are in position for operation. In operation, the centrifuge 78 rotates the suspended bowl and spool elements 80 and 82 about an axis.

In the illustrated embodiments, each circumferential flow chamber assembly multi-stage processing. A first stage separates RBC and PRP from WB. A second stage separates PC and PPP from the PRP.

While the interior of either circumferential flow chamber assembly 74 or 76 can be variously arranged, Figs. 6/7 and 8/9 show the interior of the alternative circumferential flow chambers divided into two side-by-side processing compartments 84 and 86. In use, centrifugal forces in the first compartment 84 separate whole blood into RBC and PRP. Centrifugal forces in the second processing compartment 86 separate the PRP from the first stage into PC and PPP.

In both alternative circumferential flow chambers, a first peripheral seal 88 forms the outer edge of the circumferential flow chamber assembly 74 or 76. A second interior seal 90 divides the circumferential flow chamber assembly 74 or 76 into the first processing compartment 84 and the second processing compartment 86. The second seal 90 extends generally parallel to the rotational axis of the chamber assembly 74 or 76; that is, it extends across the circumferential flow of the chamber assembly 74 or 76. The second seal 90 constitutes a longitudinal edge common to both first and second processing compartments 84 and 86.

Each processing compartment 84 and 86 serves as a separate and distinct separation chamber and will therefore be referred to as such.

In each alternative circumferential flow chamber, five ports 92/94/96/98/100 open into the compartmentalized areas formed in the processing chamber assembly 74 or 76. The ports 92/94/96/98/100 are arranged side-by-side along the top transverse edge of the respective chamber 84 and 86.

The ports 92/94/96/98/100 are all axially oriented; that is, their axes are aligned with the axis of rotation, transverse the circumferential fluid flow path within the chamber assembly 74 or 76 itself. Three ports 92/94/96 serve the first chamber 84. Two ports 98/100 serve the second chamber 86.

In both alternative circumferential flow chamber assemblies 74 and 76, an umbilicus 102 (see Fig. 14) attached to the ports 92/94/96/98/100 interconnects the first and second chambers 84 and 86 with each other and with pumps and other stationary components located outside the rotating components of the centrifuge 78.

As Fig. 11 shows, a non-rotating (zero omega) holder 104 holds the upper portion of the umbilicus 102 in a non-rotating position above the suspended spool and bowl elements 80 and 82. A holder 106 on the yoke 85 rotates the mid-portion of the umbilicus 102 at a first (one omega) speed about the suspended spool and bowl elements 80 and 82. Another holder 108 (see Fig. 12) rotates the lower end of the umbilicus 102 at a second speed twice the one omega speed (the two omega speed), at which the suspended spool and bowl elements 80 and 82 also rotate. As before stated, this known relative rotation of the umbilicus keeps it untwisted, in this way avoiding the need for rotating seals.

Using either alternative circumferential flow chamber assembly 74 or 76, the two omega speed at which the suspended spool and bowl elements 80 and 82 rotate is about 3400 RPM. Given the dimensions of the spool and bowl elements 80 and 82, 3400 RPM will develop a centrifugal force field of about 900 G's along the high-G wall 66 of the chambers 84 and 86.

A. The First Stage Processing Chamber

In the embodiment shown in Figs. 6 and 7, the first port 92 comprises the previously described PRP collection port (identified by reference numeral 72, as in Figs. 1 to 3). The second port 94 comprises the previously described WB inlet port (identified by reference numeral 68, as in Figs. 1 to 3). The third port 96 comprises the previously described RBC collection port (identified by reference numeral 70, as in Figs. 1 to 3).

A third interior seal 110 is located between the PRP collection port 72 and the WB inlet port 68. The third seal 110 includes a first region 112 that is generally parallel to the second interior seal 90, thereby extending across the circumferential WB flow path. The third interior seal 110 then bends in a dog-leg portion 114 away from the WB inlet port 68 in the direction of circumferential WB flow. The dog-leg portion 114 terminates beneath the inlet of the PRP collection port 72.

A fourth interior seal 116 is located between the WB inlet port 68 and the RBC collection port 74. The fourth seal 116 includes a first region 118 that is generally parallel to the second and third interior seals 90 and 110, thereby extending across the circumferential WB flow path. The fourth interior seal 116 then bends in a dog-leg portion 120 away from the RBC collection port 70 in the direction of circumferential WB flow. The dog-leg portion 120 extends beneath and beyond the dog-leg portion 114 of the third seal 110. It terminates near the longitudinal side edge of the first chamber 84 that is opposite to the longitudinal side edge formed by the second interior seal 90.

Together, the third and fourth interior seals 110/116 form a WB inlet passage 122 that first extends along the axis of rotation (i.e., between the first regions 112/118 of the two seals 110/116). The WB inlet passage 122 then bends to open in the direction of intended circumferential flow within the first chamber 84 (i.e., between the dog-leg portions 114/120 of the two seals 110/116).

The WB inlet passage 122 first channels WB away from the WB inlet port 68 in an axial flow path. It then channels WB circumferentially, directly into the circumferential flow path, where separation into RBC and PRP begins.

The third interior seal 110 also forms a PRP collection region 124 within the first chamber 84 (i.e., between the third seal 110 and the adjacent upper portion of the first peripheral seal 88).

Together, the fourth interior seal 116, the second interior seal 90, and the lower regions of the first peripheral seal 88 form a RBC collection passage 126 that extends first along the axis of rotation (i.e., between the second interior seal 90 and the fourth interior seal 116). The RBC collection passage 126 then bends in a circumferential path to open near the end of the intended WB circumferential flow path (i.e., between the dog-leg portion 120 of the fourth seal 116 and the lower region of the peripheral seal 88).

In the embodiment shown in Figs. 8 and 9, the first port 92 comprises the RBC collection port (identified by reference numeral 70, as in Figs. 1 to 3). The second port 94 comprises the PRP collection port (identified by reference numeral 72, as in Figs. 1 to 3). The third port 96 comprises the WB inlet port (identified by reference numeral 68, as in Figs. 1 to 3).

As Fig. 8 shows, a third interior seal 110 is located between the PRP collection port 72 and the WB inlet port 68. The seal 110 includes a first region 112 that is generally parallel to the second interior seal 90. It then bends in a dog-leg portion 114 away from the WB inlet port 68 in the direction of circumferential WB flow. The dog-leg portion 114 terminates beneath the inlet of the PRP collection port 72.

Together, the second and third interior seals 90 and 110 form a WB inlet passage 122, like the WB inlet passage 122 associated with the chamber 84 shown in Fig. 6, except in a different location within the chamber.

As Fig. 8 shows, a fourth interior seal 116 is located between the PRP collection port 72 and the RBC collection port 72 includes a first region 118 that is generally parallel to the second and third seals 90 and 110, thereby extending across the circumferential flow path. The fourth interior seal 116 then bends in a dog-leg portion 120 away from the PRP collection port 72 in the direction of circumferential WB flow. It terminates near the longitudinal side edge of the first chamber 84 that is opposite to the longitudinal side edge formed by the second interior seal 90.

Together, the fourth interior seal 116 and the upper regions of the first peripheral seal 88 form a RBC collection passage 126, like the RBC collection passage 126 shown in Fig. 6, except that it is located at the top of the chamber 84, instead of at the bottom.

As Fig. 8 shows, the third and fourth interior seals 110 and 116 together also form a PRP collection region 124 within the first chamber, like the PRP collection region 124 shown in Fig. 6.

The dynamic flow conditions within each alternative circumferential flow chamber assembly 74 or 76 are the same. These conditions direct PRP toward the PRP collection region 124 for collection through the inlet of the PRP collection port 72.

As Figs. 6 and 8 show, the WB inlet passage 122 channels WB directly into the circumferential flow path immediately next to the PRP collection region 124. Here, the radial flow rates of plasma are greatest to lift platelets free of the interface and into the PRP collection region 124.

The RBC collection passage 126 receives RBC at its open end and from there channels the RBC to the RBC collection port 70. As Figs. 6 and 8 show, the WB inlet passage 122 channels WB directly into the flow path at one end of the first chamber 84, and the RBC collection passage 126 channels RBC out at the opposite end of the flow path.

In each alternative circumferential flow chamber assembly 74 and 76 (as Figs. 7 and 9 respectively show), the low-G wall 64 of the first chamber 84 is offset toward the high-G wall 66 near the RBC collection region.

In the particular embodiments shown, the low-G wall 64 tapers into the chamber 84 in the direction of circumferential WB flow. The taper proceeds from the second interior seal 90 toward the opposite longitudinal end of the chamber. Fig. 3 shows the tapering low-G wall 64 from another perspective.

The tapering low-G wall 64 includes a stepped-up barrier 128 or dam in the region where the RBC collection passage 126 opens. As Figs. 6 and 8 show for their respective chamber assembly, the stepped-up barrier 128 extends from the low-G wall 64 across the entire chamber 84.

As Fig. 3 best shows from another perspective, the stepped-up barrier 128 extends into the RBC mass and creates a restricted passage 129 between it and the facing high-G wall 66. The restricted passage 129 allows RBC present along the high-G wall 66 to move beyond the barrier 128 for collection by the RBC collection passage 126. Simultaneously, the stepped up barrier 128 blocks the passage of the PRP beyond it, keeping the PRP within the dynamic flow conditions leading to the PRP collection region 124.

While various configurations can be used, in a preferred arrangement, the low-G wall 64 tapers about 2 mm into the chamber 74 where it joins the barrier 128. The barrier 128 extends from there at about a 45 degree angle toward the high-G wall 66, forming a raised planar surface. The passage 129 formed between the planar surface and the high-G wall 66 is about 1 mm to 2 mm in radial depth and about 1 mm to 2 mm in circumferential length.

As previously described (and as Fig. 3 shows), the configuration of the low-G wall 64 creates a swift counterflow of plasma from the RBC collection region toward the PRP collection region 124.

The desired contours for the low-G wall 64 of the alternative chamber assemblies 74 and 76 can be preformed on the exterior surface of the spool element 82. In the illustrated embodiment, the interior surface of the bowl element 82 is isoradial with respect to the rotational axis.

Also in both alternative embodiments (as Figs. 6 and 8 show), the dog leg portion 120 of the RBC collection passage 126 is tapered. Due to the taper, the passage 126 presents a greater cross section where it opens into the chamber 84 than it does where it joins the axial first region 118 of the RBC collection passage 126. Fig. 3 shows this taper from another perspective. In the illustrated and preferred embodiment, the dog leg portion 120 tapers from a width of about 6.35mm (1/4 inch) to 3.175mm (1/8 inch).

The taper of the dog leg portion 120 is preferably gauged relative to the taper of the low-G wall 64 to keep the cross sectional area of the RBC collection passage 126 substantially constant. This keeps fluid resistance within the passage 126 relatively constant, while maximizing the available separation and collection areas outside the passage 126. The taper of the dog leg portion 120 also facilitates the removal of air from the passage 126 during priming.

As Figs. 6 and 8 best show, a ramp 130 extends from the high-G wall 66 across the PRP collection region 124 in each alternative chamber assembly 74 and 76. As Fig. 14 shows from another perspective, the ramp 130 forms a tapered wedge that restricts the flow of fluid toward the PRP collection port 72. As Fig. 15 shows, the ramp 130 forms a constricted passage 131 along the low-G wall 64, along which the PRP layer extends.

In the illustrated embodiment (see Fig. 12), a hinged flap 132 extends from and overhangs a portion of the spool element 82. The flap 132 is preformed to present the desired contour of the ramp 130.

When flipped down (as Fig. 12 shows in solid lines), the flap 132 is sandwiched between the chosen chamber assembly 74/76 and the surrounding bowl element 80. The flap 132 presses against the adjacent flexible wall of the chamber assembly 74/76, which conforms to its contour to form the ramp 130 within the chamber 84.

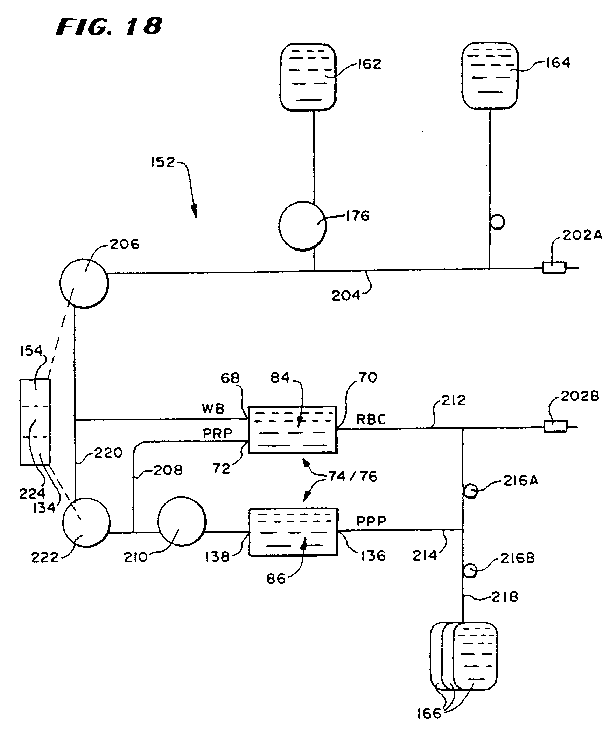

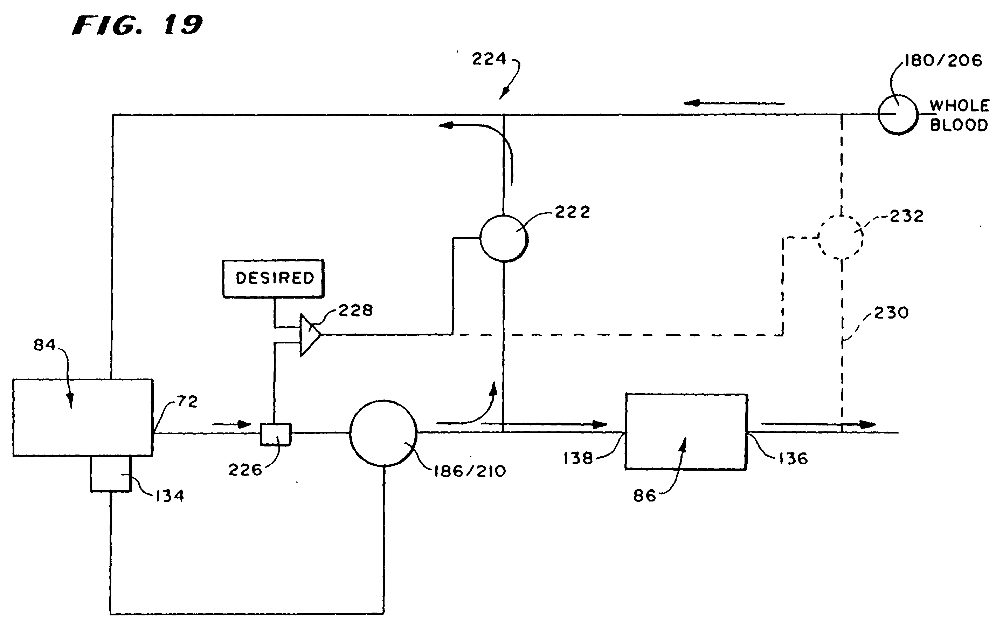

As shown diagrammatically in Figs. 15A to C, the ramp 130 diverts the fluid flow along the high-G wall 66. This flow diversion changes the orientation of the interface 26 between the RBC (shown shaded in Figs. 15A/B/C) and the PRP (shown clear in Figs. 15A/B/C) within the PRP collection region 124. The ramp 130 displays the interface 26 for viewing through a side wall of the chamber assembly 74/76 by an associated interface controller 134 (that Figs. 18 and 19 show).

As will be described in greater detail later, the interface controller 134 monitors the location of the interface 26 on the ramp 130. As Figs. 15A/B/C show, the position of the interface 26 upon the ramp 130 can be altered by controlling the relative flow rates of WB, the RBC, and the PRP through their respective ports 68/70/72. The controller 134 varies the rate at which PRP is drawn from the chamber 84 to keep the interface 26 at a prescribed location on the ramp 26 (which Fig. 15B shows), away from the constricted passage 131 that leads to the PRP collection port 72.

The ramp 130 and associated interface controller 134 keep RBC, white blood cells, and lymphocytes present in the interface 26 from entering the PRP collection port 72. The collected PRP is thereby essentially free of the other cellular components present in the interface 26.

B. The Second Stage Processing Chamber

In the embodiment of the chamber assembly shown in Figs. 6/7, the fourth port 98 constitutes a PPP collection port 136, and the fifth port 100 constitutes a PRP inlet port 138. In the embodiment shown in Figs. 8/9, the opposite is true; the fourth port 98 constitutes the PPP inlet port 138, and the fifth port 100 constitutes the PPP collection port 136.

In each chamber assembly 74/76, the umbilicus 102 connects the PRP collection port 72 of the first chamber 84 with the PRP inlet port 138 of the associated second chamber 86. The second chamber 86 thereby receives PRP from the first chamber 84 for further separation into PPP and PC. The umbilicus 102 conveys separated PPP from the second chamber 86 through the associated PPP collection port 136. In each assembly 74/76, the PC remains behind in the second chamber 86 for later resuspension and collection.

In the alternative embodiments shown in Figs. 6/7 and 8/9, a fifth interior seal 140 extends between the PRP inlet port 138 and the PPP collection port 136. The fifth seal 140 includes a first region 142 that is generally parallel to the second seal 90, thereby extending across the circumferential flow path. The fifth interior seal 140 then bends in a dog-leg portion 144 away from the PRP inlet port 138 in the direction of circumferential PRP flow within the second chamber 86.

The dog-leg portion 144 terminates near the longitudinal side edge of the second chamber 86 that is opposite to the longitudinal side edge formed by the second interior seal 90.

In the Figs. 6/7 embodiment, the fifth interior seal 140, the second interior seal 90, and the lower regions of the first peripheral seal 88 together form a PPP collection passage 146 that extends first along the axis of rotation (i.e., between the second interior seal 90 and the fifth interior seal 140) and then bends in a circumferential path to open near the end of the intended PRP circumferential flow path (i.e., between the dog-leg portion 144 of the fifth seal 140 and the lower region of the peripheral seal 88). The PPP collection passage 146 receives PPP at its open end and from there channels the PPP to the PPP collection port 136.

In the Figs. 8/9 embodiment, a similar PPP collection passage 146 is formed between the fifth interior seal 140 and the upper region of the peripheral seal 88.

In each alternative circumferential flow chamber assembly 74/76, PRP entering the second chamber 86 via the PRP inlet port 138 is caused to flow first in an axial path from the axially oriented PRP inlet port 138 alongside the axially extending fifth seal 140. The flow direction of the PRP then turns to a circumferential path away from the fifth seal 140 toward the opposite longitudinal side edge.

The centrifugal forces generated during rotation of the chamber separate the PRP into PC and PPP. The more dense PC separate out into a layer that extends along the high-G wall 66. The less dense PPP is displaced toward the low-G wall 64 for collection through the PPP collection passage 146.

The inventor has discovered that the introduction of PRP along an axial flow path parallel to the axis of rotation into a circumferential flow path about the axis of rotation creates a non-turbulent vortex region 148, called a Taylor column, at the outlet of the PRP inlet port 138, as Fig. 16 shows.

The vortex region 148 circulates about an axis that is aligned with the axis of the PRP inlet port 138. The vortex region 148 stretches from the outlet of the port 138 longitudinally across the circumferential flow path of the chamber 86. As Fig. 16 shows, the vortex region 148 circulates the PRP about its axis and directs it into the desired circumferential flow path within the chamber 86.

Within the vortex region 148, axial flow velocity decreases in a generally linear fashion across the circumferential flow path of the chamber 86. This occurs as the axial flow of fluid entering the chamber 86 perfuses uniformly into a circumferential flow entering the separation zone.

A similar vortex region 148 forms at the opposite longitudinal end of the second chamber 86 at the entrance to the PPP collection passage 146, as Fig. 16 also shows.

The vortex region 148 created at the outlet of the PRP inlet port 138 uniformly disperses PRP in the desired circumferential flow path into the centrifugal field. This maximizes the exposure of the entering PRP to the effects of the centrifugal field across the effective surface area of the second chamber 86. Maximum possible separation of PC from the entering PRP results.

It should be noted that similar vortex region 148 flow conditions are formed in the first chamber 84 as well, where fluid either enters or leaves the established circumferential flow path through an axial flow path. As Fig. 16 shows, a vortex region 148 condition thereby forms at the entrance of the WB inlet passage 122. Another vortex region 148 condition forms at the opposite longitudinal end at the entrance of the RBC collection passage 126.

In both alternative chamber assemblies 74/76 (as Figs. 7 and 9 show), the low-G wall 64 preferably tapers into the second chamber 86 in the direction of circumferential PRP flow. The taper proceeds from the second interior seal 90 toward the opposite longitudinal end of the second chamber 86.

Also in both alternative chamber assemblies 74/76 (as Figs. 6 and 8 show), the circumferential leg of the associated PPP collection passage 146 is tapered. Due to the taper, the leg presents a greater cross section where it opens into the second chamber than it does where it joins the axial portion of the PPP collection passage 146. In the illustrated and preferred embodiment, the leg tapers from a width of about 6.35 mm to 3.175mm (1/4 inch to 1/8 inch).

As with the taper of the dog leg portion 120, the taper of the circumferential leg of the PPP collection passage 146 is preferably gauged relative to the taper of the low-G wall 64 to keep the cross sectional area of the PPP collection passage 146 substantially constant. This keeps fluid resistance within the passage 146 relatively constant. The taper of the circumferential leg of PPP collection passage 146 also facilitates the removal of air from the passage 146 during priming.

The dimensions of the various regions created in the processing chamber can of course vary according to the processing objectives. Table 1 shows the various dimensions of a representative embodiment of a processing chamber of the type shown in Figs. 6/7 or 8/9. Dimensions A through F referenced in Table 1 are identified for their respective chamber assemblies in Figs. 6 and 8.

TABLE 1

Overall length (A): 19-1/2 inches

Overall height (B): 2-13/16 inches

First Stage Processing Chamber

Length (C): 10-1/8 inches

Width (D): 2-3/8 inches

Maximum Radial Depth in Use: 4 mm

Second Stage Processing Chamber

Length (E): 8-13/16 inches

Width (F): 2-3/8 inches

Maximum Radial Depth in Use: 4 mm

Port Spacing

(center line to center line): 3/8 inch

NB: one inch = 25.4mm.

## II. SYSTEMS USING THE ENHANCED YIELD CIRCUMFERENTIAL FLOW CHAMBER FOR PLATELET SEPARATION AND COLLECTION

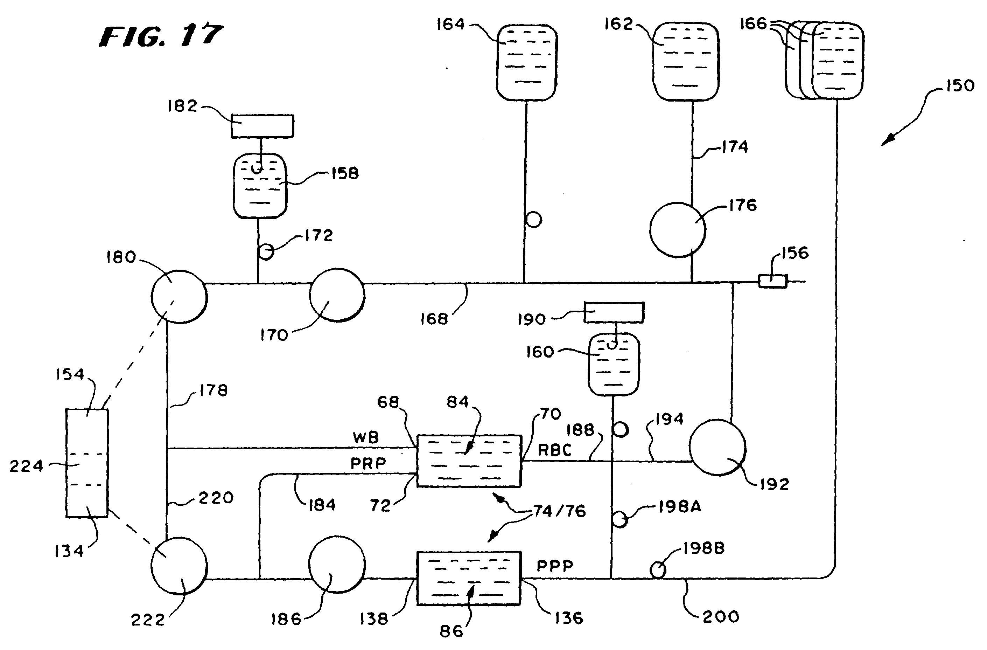

The two stage circumferential flow chambers shown in either Figs. 6/7 or Figs. 8/9 can be used to do continuous platelet collection. The chambers can be used in associated either with a system 150 that employs one phlebotomy needle (as Fig. 17 shows) or with a system 152 that employs two phlebotomy needles (as Fig. 18 shows). In each system 150 and 152, an associated processing controller 154 automates the collection procedure to the fullest extent possible.

### A. Single Needle Enhanced Yield Platelet Collection System

The platelet collection system 150 shown in Fig. 17 employs one, single lumen phlebotomy needle 156. Fig. 11 generally depicts this single needle system 150 when mounted for use on the centrifuge 78.

The processing controller 154 operates the single needle system 150 in a draw cycle and a return cycle.

During the draw cycle, the controller 154 supplies the donor's WB through the needle 156 to a chosen one of the processing chamber assemblies 74/76. There, the WB is centrifugally separated into RBC, PC, and PPP.

During the return cycle, the controller 154 returns RBC and PPP to the donor through the needle 156, while separation within the chosen processing chamber assembly 74/76 continues without interruption. The harvested PC is retained for long term storage. If desired, all or some PPP can be retained for storage, too.

The system 150 includes a draw reservoir 158, which pools a quantity of the donor's WB during the draw cycle. The system 150 also includes a return reservoir 160, where a quantity of RBC collect for periodic return to the donor during the return cycle.

Processing containers associated with the system 150 include a container 162 that holds anticoagulant for use during the procedure and a container 164 that holds saline solution for use in priming and purging air from the system 150 before the procedure. The system further includes collection containers 166 for receiving PC (and optionally PPP) for storage.

When the controller 154 operates the system 150 in the draw cycle, a first branch 168 directs WB from needle 156 to the draw reservoir 158, in association with the draw pumping station 170 and a clamp 172. An auxiliary branch 174 delivers anticoacjulant to the WB flow in association with an anticoagulant pumping station 176.

A second branch 178 conveys the WB from the draw reservoir 158 to the WB inlet port 68 of the chosen processing chamber assembly 74/76, in association with the WB inlet pumping station 180. The draw pumping station 170 operates at a higher flow rate (at, for example, 100 ml/min) than the WB inlet pumping station 180, which operates continuously (at, for example, 50 ml/min).

The processing controller 154 includes a first scale 182 that monitors the weight volume of WB collected in the draw reservoir 158. The first scale 182 intermittently operates the draw pumping station 170 to maintain a desired weight volume of WB in the draw reservoir 158.

Once the desired volume of WB is present in the draw reservoir 158, the WB inlet pumping station 180 operates to continuously convey WB into the chosen processing chamber assembly 74/76.

The draw pumping station 170 continues to operate periodically during the draw cycle in response to the scale 182 to maintain the desired weight volume of WB in the draw reservoir 158.

The WB enters the first stage chamber 84, where it is separated into RBC and PRP. This separation process has already been described.

A third branch 184, in association with the plasma pumping station 186, draws the PRP from the PRP collection port of the first processing chamber 84. The third branch 184 conveys the PRP to the PRP inlet port 138 of the second processing chamber 86. There, the PRP is further separated into PC and PPP. This separation process has already been described.

As will be described in greater detail later, the processing controller 154 monitors the location of the interface on the ramp 130 via the interface controller 134. The controller 154 operates the plasma pumping station 186 to keep the maximum rate of the variable plasma pumping station 186 (for example, 25 ml/min) less than the WB inlet pumping station 180.

A fourth branch 188 conveys the RBC from the RBC collection port 74 of the first stage processing chamber 84. The fourth branch 188 leads to the return reservoir 160.

The processing controller 154 includes a second scale 190 that monitors the weight volume of RBC in the return reservoir 160. When a preselected weight volume exists, the controller 154 shifts the operation of the system 150 from its draw cycle to its return cycle.

In the return cycle, the controller 154 stops the draw pumping station 170 and starts a return pumping station 192. A fifth branch 194 associated with the return pumping station 192 conveys RBC from the return reservoir 160 to the needle 156.

Meanwhile, while in the return cycle, the controller 154 keeps the WB inlet pumping station 180 and plasma pumping station 186 in operation to continuously process the WB pooled in the draw reservoir 158 through the first processing chamber 84.

During both draw and return cycles, PRP enters the PRP inlet port 138 of the second stage processing chamber 86. The PPP exits the PPP collection port 136 of the second stage processing chamber through a sixth branch 196 and into the return reservoir 160, joining the RBC there pooled.

Alternatively, by closing the clamp 198A and opening the clamp 198B, the PPP can be conveyed through a seventh branch 200 to one or more collection containers 166.

After a procedure, the PC collected within the second processing compartment 86 is transferred via the seventh branch 200 to one or more collection containers 166 for storage.

B. <u>Double Needle Platelet Collection System</u>

The platelet collection system 152 shown in Fig. 18 employs two single lumen phlebotomy needles 202A and 202B to obtain generally the same processing results as the single needle system 150 shown in Fig. 17. Elements common to both systems 150 and 152 are assigned the same reference numeral.

The associated processing controller 154 operates the system 152 in a continuous cycle, during which the donor's WB is continuously supplied through the needle 202A to the chosen processing chamber assembly 74/76 for separation into RBC, PC, and PPP, while RBC and PPP are continuously returned to the donor through the needle 202B.

As in the single needle system 150, the harvested PC is retained for long term storage. If desired, all or some PPP can be diverted from the donor for storage.

As in the single needle system 150, the processing containers associated with the double needle system 152 include a container 162 that holds anticoagulant and a container 164 that holds saline solution for use in priming and purging air from the system 152.

The system 152 also includes similar collection containers 166 for receiving PC (and optionally PPP) for storage.

Under the control of the controller 154, a first branch 204 directs WB from the needle 202A to the WB inlet port 68 of the first stage processing chamber 84, in association with the WB inlet pumping station 206, which operates continuously at, for example, 50 ml/min. An auxiliary branch 174 delivers anticoagulant to the WB flow in association with an anticoagulant pumping station 176.

The WB enters and fills the first processing chamber 84 in the manner previously described, where centrifugal forces generated during rotation of the chosen chamber assembly 74/76 separate the WB into RBC and PRP.

A second branch 208, in association with the plasma pumping station 210, draws the PRP layer out the PRP collection port 72 of the first stage processing chamber 84, conveying the PRP to the PRP inlet port 138 of the second stage processing chamber 86, where it undergoes further separation into PC and PPP.

The processing controller 154 monitors the location of the interface on the ramp 130 and varies the speed of the plasma pumping station 210 (using the interface controller 134, to be described later in greater detail) to keep the interface 26 at a prescribed location on the ramp 130. As before described, the controller 154 keeps the maximum rate of the variable plasma pumping station 210 (for example, 25 ml/min) less than the WB inlet pumping station 206.

A third branch 212 conveys the RBC from the RBC collection port 70 of the first stage processing chamber 84. The third branch 212 leads to the needle 202B.

The PPP exits the PPP collection port 136 of the second stage processing chamber 86 through a fourth branch 214, joining the third branch 212 (carrying RBC) leading to the needle 202B. Alternatively, by closing the clamp 216A and opening the clamp 216B, the PPP can be conveyed through a fifth branch 218 to one or more collection containers 166.

After a procedure, the PC collected within the second processing compartment 86 is transferred via the fifth branch 218 to one or more collection containers 166 for storage.

C. <u>Enhancing Platelet Separation by Plasma Recirculation</u>

Both single and double needle systems 150 and 152 (shown in Figs. 17 and 18 respectively) include a recirculation branch 220 and an associated recirculation pumping station 222. The processing controller 154 has a recirculation control system 224 that operates the pumping station 222 to convey a portion of the PRP exiting the PRP collection port 72 of the first processing compartment 84 for remixing with the WB entering the WB inlet port 68 of the first processing compartment 84.

The control system 224 can control the recirculation of PRP in different ways.

As Fig. 19 shows, the recirculation control system 224 includes a sensor 226 that senses the flow rate at which PRP exits the first processing compartment 84, under the control of pumping station 186 (for the single need system 150) or pumping station 210 (for the double needle system 152). As will be described in greater detail, this flow rate is itself controlled by the interface controller 134.

The recirculation control system 224 employs a comparator 228 to compare the sensed PRP flow rate to an established desired flow rate. If the sensed rate is less than the desired flow rate, the comparator 228 sends a signal to increase rate at which the recirculation pumping station 222 operates. And, if the sensed rate is more than the desired flow rate, the comparator 228 sends a signal to decrease the rate at which the recirculation pumping station 222 operates. In this way, the comparator 228 maintains the PRP flow rate at the desired rate.

The desired PRP output rate is preselected to create within the first compartment 84 the processing conditions which maximize the concentration of platelets in the PRP stream.

The desired rate of recirculation is based upon the radial flow rate of plasma desired in the region where PRP is collected.

According to another aspect, the pumping rate of the recirculation pump 222 is maintained as a percentage (%RE) of the pumping rate of the whole blood inlet pump 180/206, governed as follows:

$$\%_{RE} = K * Hct - 100$$

where:

Hct is the hematocrit of the donor's whole blood, measured before donation, and

K is a dilution factor that takes into account the volume of anticoagulant and other dilution fluids (like saline) that are added to the donor's whole blood before separation.

According to this aspect the pumping rate of the recirculation pump 222 is maintained at the predetermined percentage ($\%_{RE}$) of the pumping rate of the whole blood inlet pump 180/206 to maintain a surface hematocrit of about 30% to 35 % in the entry region $R_e$. The preferred surface hematocrit in the entry region $R_e$, is believed to be about 32%.

Keeping the surface hematocrit in the entry region $R_e$ in the desired range provides optimal separation of RBC from PRP, thereby optimizing the radial flow of plasma in this region. If the surface hematocrit exceeds the predetermined range, radial plasma flow in the entry region $R_e$, decreases. If the surface hematocrit falls below the predetermined range, the radial flow of PRP increases enough to sweep small RBC's and white blood cells into the PRP.

The value of the dilution factor K can vary according to operating conditions. The inventor has determined that K=2.8, when ACD anticoagulant is added to constitute about 9% of the entry whole blood volume, and a saline dilution fluid is added in an amount representing about 4% of donor body volume (i.e., 200 ml saline for 5000 ml in body volume).

In an alternate arrangement (shown in phantom lines in Fig. 19), the recirculation control system 224 recirculates PPP, instead of PRP, based upon $\%_{RE}$, as determined above.

In this arrangement, the system 224 uses a recirculation branch 230 and associated pumping station 232 located downstream of the second processing compartment 86. The comparator controls the pumping station 232 in one of the same manners just described to mix PPP exiting the second compartment 86 with the incoming WB entering the first compartment 84.

By mixing PRP (or PPP) with the WB entering the first processing compartment 84 to control surface hematocrit in the entry region $R_e$, the velocity at which red blood cells settle toward the high-G wall 66 in response to centrifugal force increases. This, in turn, increases the radial velocity at which plasma is displaced through the interface 26 toward the low-G wall 64. The increased plasma velocities trough the interface 26 elute platelets from the interface 26. As a result, fewer platelets settle on the interface 26.

Example 1

A study evaluated a two stage separation chamber 74 like that shown in Fig 6 in a platelet collection procedure on a healthy human donor. The chamber 74 was part of a double needle system 152, like that shown in 28. The system 152 recirculated PRP in the manner shown in Fig. 18 to obtain a hematocrit of 32.1% in the PRP collection region 124 of the chamber 74.

In this study, the low-G wall 64 of the first stage chamber 84 was not tapered in the direction of circumferential flow from the PRP collection region 124. The low-G wall 64 was isoradial along the circumferential flow path in the first stage chamber 84, except for the presence of a RBC barrier 128, which stepped into the chamber across the RBC collection passage, as shown in Fig. 7. The low-G wall 64 was isoradial along the entire circumferential flow path of the second chamber 86.

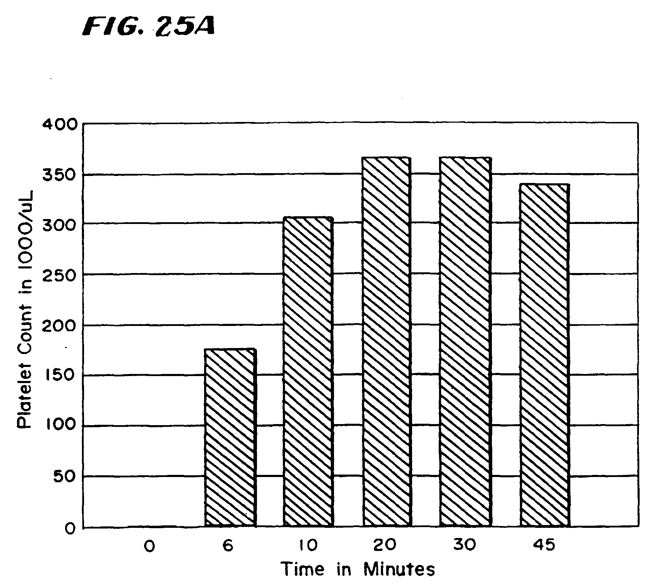

Fig. 25A shows the platelet count sampled in the PRP (in 1000 platelets per μL) over time during the 45 minute procedure. As there shown, after a run time of 6 minutes, the platelet count was 173; after 10 minutes, the platelet count was 304; and after 20 minutes, the platelet count stabilized at 363.

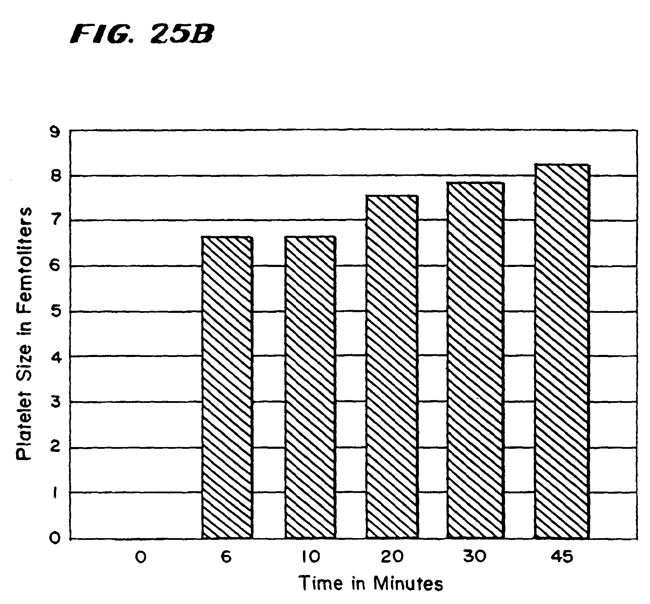

Fig. 25B shows the physical size of the platelets collected in the PRP in terms of mean platelet volume (in femtoliters) sampled during the procedure. As there shown, after a run time of 6 minutes, the mean platelet size was 6.6; after 20 minutes, the mean platelet size rose to 7.5; and at the end of the procedure, the mean platelet size was 8.2. A size distribution study of the PC collected showed that about 3 % of the platelets collected were larger than 30 femtoliters (i.e., were very large platelets).

The platelet transfer efficiency in the first stage chamber 84 (i.e., the percentage of available platelets entering the first stage chamber 84 that were ultimately collected in the PRP) was 93.8 %. In other words, the first stage chamber 84 failed to collect only 6.2% of the available platelets in the first stage chamber 84.

The platelet transfer efficiency in the second stage chamber 86 (i.e., the percentage of available platelets in the PRP entering the second stage chamber 86 that were ultimately collected as PC) was 99%. In other words, the second stage chamber 86 failed to collect only 1 % of the platelets present in the PRP in the second stage chamber 86.

The overall platelet collection efficiency of the chamber was about 81%, meaning that about 81% of the platelets in the whole blood processed were ultimately collected. This is a significantly higher amount than conventional processing can provide. In comparison, the comparable overall platelet collection efficiency for two stage CS-3000® Centrifuge chamber is about 50%. (The CS-3000 centrifuge chamber is a blood separation chamber made by Baxter Healthcare Corporation.)

This study demonstrates the increased separation efficiencies that result from chambers that embody features of the invention.

Example 2

Another study evaluated a two stage separation chamber like that in Example 1 in a platelet collection procedure on a healthy human donor. As in Example 2, a double needle system was used. The system recirculated PRP to obtain an inlet hematocrit of 34.3%.

In this study, the low-G wall 64 of the first stage chamber 84 was tapered in the direction of circumferential flow from the PRP collection region 124, like that shown in Fig. 7. The low-G wall 64 also included RBC barrier 128 like that shown in Fig. 7. The low-G wall 64 was also tapered along the entire circumferential flow path of the second chamber 86.

Fig. 26A shows the platelet count sampled in the PRP (in 1000 platelets per uL) over time during the 45 minute procedure. As there shown, a platelet count of 300 was achieved in the first 5 minutes of the procedure. The platelet count peaked at 331 after 21 minutes. At the end of the procedure, the platelet count was 302.

Fig. 26B shows the physical size of the platelets collected in the PRP in terms of mean platelet volume (in femtoliters) sampled during the procedure. As there shown, after a run time of only 5 minutes, the mean platelet size was 8.6, where it virtually remained throughout the rest of the procedure. A size distribution study of the PC collected showed that about 8.5% of the platelets collected were larger than 30 femtoliters.

The second study also experienced greater collection efficiencies.

The platelet transfer efficiency in the first stage chamber 84 (i.e., the percentage of available platelets that were ultimately collected in the PRP) was 99.2%. In other words, the first stage chamber 84 failed to collect less than 1% of the available platelets.

The platelet transfer efficiency in the second stage chamber 86 (i.e., the percentage of available platelets in the PRP that were ultimately collected as PC) was 99.7%. In other words, the second stage chamber 86 collected nearly all the platelets present in the PRP.

The overall platelet collection efficiency of the chamber was 85.3 %.

This study further demonstrates the enhanced separation efficiencies that the invention can provide.

This study also shows the effect that the tapered low-G wall has in freeing greater number of platelets into the PRP stream. The effect is virtually immediate. After only 5 minutes in the second study, the platelet count was comparable to that encountered after 10 minutes in the first study.

This study also demonstrates the effect that the tapered low-G wall has in freeing larger platelets into the PRP stream. The effect, too, is virtually immediate. After the first 5 minutes of the procedure, the mean platelet size was comparable to that encountered after 30 minutes in the second study, which means that the larger platelets were already being collected. There were nearly 3 times more platelets of very large physical size (i.e., over 30 femtoliters) collected in the second study than in the first study.

III. INTERFACE CONTROL SYSTEMS FOR THE ENHANCED YIELD CIRCUMFERENTIAL FLOW CHAMBERS

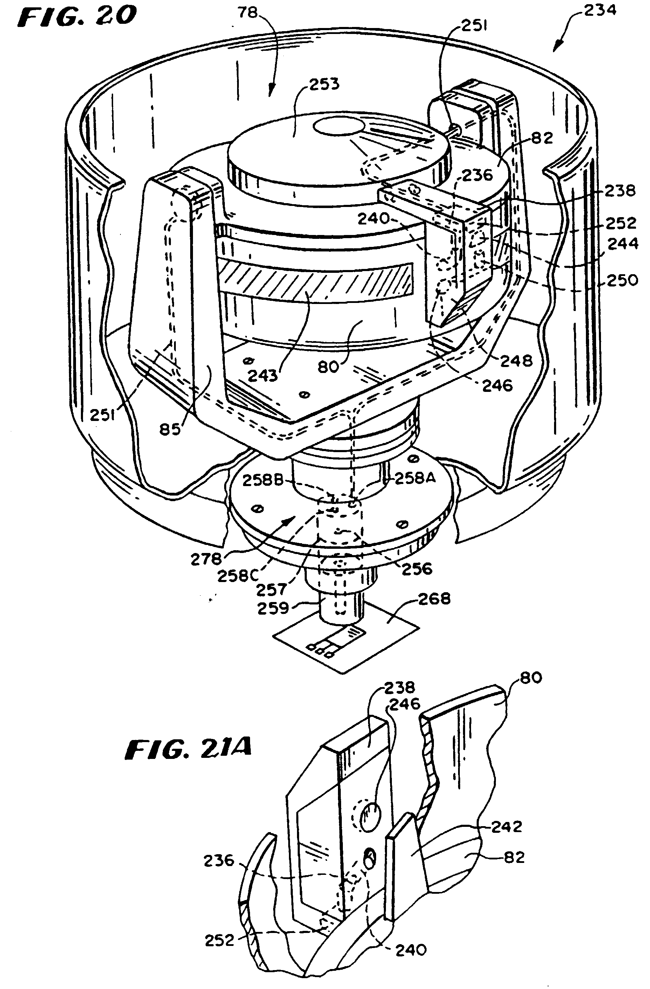

Figs. 20 to 24 show the details of an alternative interface control system 234, which can be used in association with either the single or double needle systems 150 or 152 previously described.

The interface control system 234 mounts the element that actually views the interface on a rotating element of the centrifuge. The system 234 relies upon a time pulse signal to determine the location of the interface.

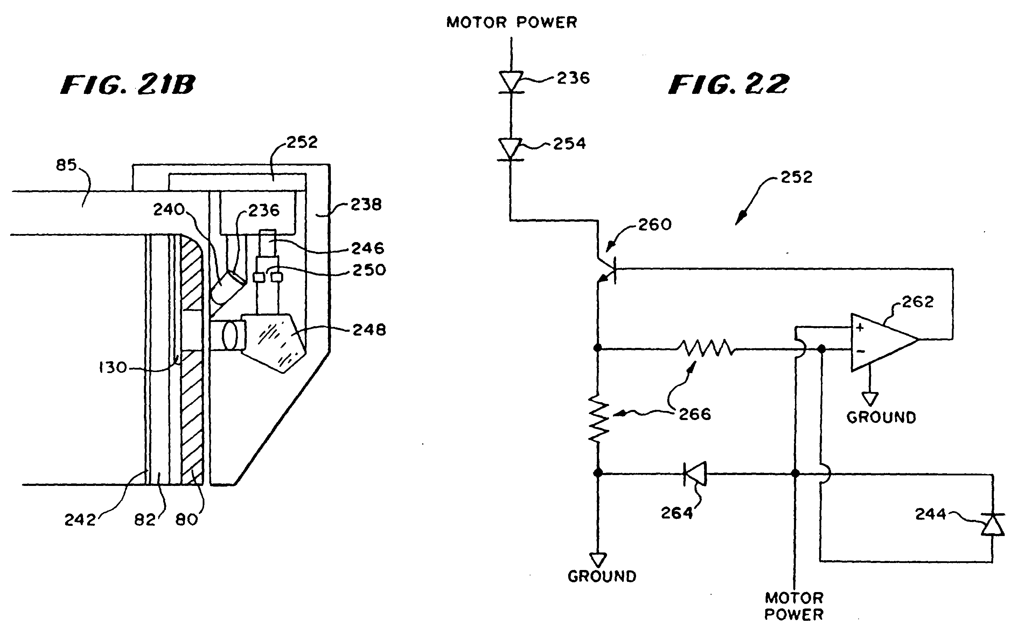

As Figs. 20 and 21 A/B show, the interface control system 234 includes a light source 236 mounted on the yoke 85 of the centrifuge 78. The source 236 emits light that is absorbed by RBC. The control system 234 also includes a light detector 244 mounted next to the light source 236 on the yoke 85.

As Fig. 20 shows, a viewing head 238 carries both the light source 236 and the light detector 244 for rotation on the yoke 85. As previously described, the yoke 85 rotates at a one omega speed, carrying the viewing head 238 with it. At the same time, the spool and bowl assemblies 80 and 82 carried by the yoke 85 rotate at a two omega speed.

In the illustrated and preferred embodiment, the viewing head 238 also serves as a counterweight for the umbilicus holder 106 that the yoke 85 also carries (also see Figs. 10 and 11).

In the illustrated and preferred embodiment, the light source 236 includes a red light emitting diode, of course, other colors, like green, could be used. In this arrangement, the light detector 244 comprises a PIN diode detector.

An optical pathway 240 directs light from the source diode 236 out onto the rotating bowl assembly 80 (see Fig. 21B). In the illustrated embodiment, the bowl assembly 80 is transparent to the light emitted by the source diode 236 only in the region where the bowl assembly 80 overlies the interface ramp 130.

The remainder of the bowl assembly 80 that lies in the path of the viewing head 238 carries a light reflecting material 243. This differentiates the reflective properties of the interface region of the bowl assembly 80 from those of the remainder of the bowl assembly 80. The material 243 could be light absorbing and serve the same purpose.

Alternatively, the source diode 236 could be gated on-and off with the arrival and passage of the interface region of the bowl assembly 80 relative to its line of sight.

The interface ramp 130 carried by the spool assembly 82 is made of a light transmissive material. The light from the source diode 236 will thus pass through the transparent region of the bowl assembly 80 and the ramp 130 every time the rotating bowl assembly 80 and viewing head 238 align.

The spool assembly 82 also carries a light reflective material 242 on its exterior surface behind the interface ramp 130 (see Fig. 26). The material 242 reflects incoming light received from the source diode 236 out through the transparent region of the bowl assembly 80. The intensity of the reflected light represents the amount of light from the source diode 236 that is not absorbed by the RBC portion of the interface region.

The light detector 244 carried in the viewing head 238 receives the reflected light through an optical pathway. In the illustrated embodiment (see Fig. 21B), the optical pathway includes a lens 246, a penta prism 248, and an aperture 250.

In the illustrated embodiment, the lens 246 is about 9 mm in diameter, with the focal length of about 9 mm. In this arrangement, the lens 246 forms a real image with a magnification of about three. Alternatively, the real image could be made smaller to provide a better depth of field.

The aperture 250 is preferably small (about 0.75 mm in diameter) to allow only a small portion of the real image to reach the detector 244. The preferred viewing field of the detector 244 is therefore small, i.e., preferably on the order of about .25 mm in diameter.

The system 234 further includes a data link 278 for transmitting light intensity signals from the rotating viewing head 268 to an interface control circuit 270 on the stationary frame of the centrifuge. In the illustrated embodiment, the data link is optical in nature. Alternatively, slip rings could be used to transmit the light intensity signals as voltage or current signals.

The optical data link 278 includes a second light source 254. The second light source 254 is carried within the confines of a hollow light conduction passage 256 within the one omega drive shaft 257.

The optical data link 278 further includes a second light detector 268. The second detector 268 is carried on the non-rotating (i.e., zero omega) base of the centrifuge below the hollow one omega drive shaft 257. Light from the second light source 254 passes through the passage 256 and a collimating sleeve 259 to fall upon the second detector 268. Like the first detector 244, the second detector 268 can comprise a PIN diode detector.

The second light source 254 comprises at least one red light emitting diode carried within the passage 256 of the one omega shaft 257. Of course, other colors, like green, could be used.

In the illustrated embodiment (see Fig. 20), the second light source 254 includes three light emitting diodes 258 A/B/C arranged at 120 degree circumferentially spaced intervals within the passage 256. This arrangement minimizes interference due to misalignment between the second light source 254 and the second detector 268. In an alternative arrangement, the light intensity signal from the second detector 268 can be electronically filtered to eliminate interference signals caused by misalignment.

The optical data link 278 also includes an intensity control circuit 252 carried onboard the viewing head 238. The intensity control circuit 252 adjusts the input to the source diode 236 so that the intensity of light hitting the detector 244 remains constant.

The intensity control circuit 252 also connects the second light source 254 in series to the first mentioned light source 236. Thus, as the intensity control circuit 252 adjust the input to the first light source 236, it will also instantaneously adjust the input to the second light source 254. Thus the intensity of the light emitted by the source 254 is proportional to the intensity of light emitted by the source 236.

As Fig. 20 shows, the system 234 delivers electrical power to its rotating components through wires 251. The same wires 251 deliver power to the electric motor 253 that rotates the spool and bowl assemblies 80 and 82.

Fig. 22 shows a representative embodiment for the intensity control circuit 252. As shown, the control circuit 252 includes a transistor 260 that controls current flow to the series-connected first and second light sources 236 and 254.

The emitter of the transistor 260 is coupled to an amplifier 262. One amplifier input is coupled to the light detector 244 carried within the yoke viewing head 238. Another amplifier input is coupled to a reference diode 264. The circuit 252 also includes conventional current limiting resistors 266 to protect the light emitting diodes of the sources 236 and 254.

As the intensity of light hitting the detector 244 decreases, the output of the amplifier 262 increases. The transistor 260 conducts more current. The intensities of the first and second light sources 236 instantaneously increase by equal or otherwise proportional amounts.

Likewise, as the intensity of light hitting the detector 244 increases, the output of the amplifier 262 decreases. The transistor 260 conducts less current. The intensities of the first and second light sources 236 instantaneously decrease by equal or proportional amounts.

As Fig. 23A shows, the interface control circuit 270 converts the sensed light intensity output of the second detector 268 to amplified voltage signals. A conventional waveshaping circuit converts the amplified voltage signals to square wave time pulses.

From the time pulses, the interface control circuit 270 derives the physical dimension of the interface (measured in inches). The interface control circuit 270 then generates a pump control signal based upon any differences between the derived interface dimension and a desired interface dimension.

As Fig. 23A shows, the first detector 244 will view fully reflected light, free of diminution at a fixed intensity $1_1$, during the period the reflective bowl material 243 and the viewing head 238 are in alignment. The second detector 268 will also view light at a fixed intensity $1_2$ generated by the second light source 254 during this period.

As the transparent interface region of the bowl assembly 80 comes into alignment with the viewing head 238, red blood cells displayed on the interface ramp 130 will enter the optical path of the viewing head 238.

The red blood cells absorb the light from the first light source 236. This absorption reduces the previously viewed intensity of the reflected light. With decreasing light intensity sensed, the control circuit 252 instantaneously increases the input to both first and second light sources 236 and 254 to maintain a constant light intensity at the first detector 244.

Under the control of the circuit 252, both light sources 236 and 254 will become brighter, assuming a new intensity level while the red blood cell band of the interface pass past the viewing head 238.

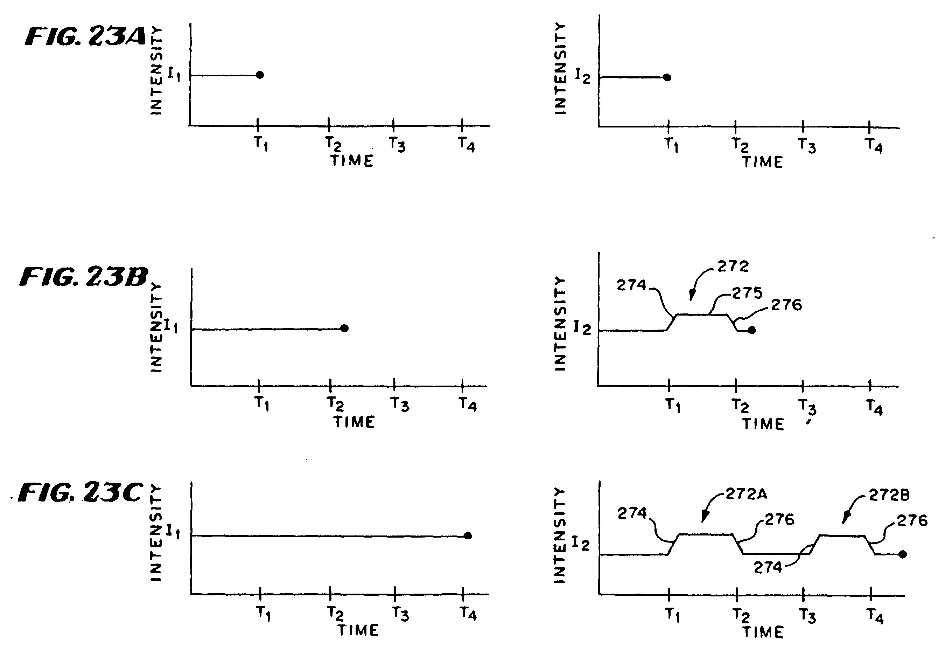

As Fig. 23B shows, the first detector 244 will not sense this relative increase in intensity over time, because the control circuit 252 instantaneously maintains the intensity $1_1$, viewed by the first detector 244 constant. However, the second detector 268 will sense this relative increase in intensity $1_2$ over time.

As Fig. 23B shows, the second detector 268 generates an increasing intensity output signal $1_2$. The interface control circuit 270 converts the increasing intensity signal into the leading edge 274 of the square pulse 272 shown in Fig. 28B. This event marks the beginning time ($T_1$) of the pulse 272.

Eventually, the intensity signal will stabilize, as the most dense region of the red cell band of the interface enters the optical path of the viewing head 238. The interface control circuit 270 converts the stabilized intensity signal into the plateau 275 of the square pulse 272 shown in Fig. 23B.

When the red cell band of the interface leaves the optical path of the viewing head 238, the first detector 244 will again view fully reflected light from the reflective bowl material 243. With increasing light intensity sensed, the control circuit 252 will instantaneously decrease the input to both first and second light sources 236 and 254 to maintain a constant light intensity at the first detector 244.

Again, the first detector 244 will not see this relative decrease m intensity over time, because the control circuit 252 instantaneously maintains the intensity $1_1$ viewed by the first detector 244 constant. However, the second detector 268 will sense this relative decrease in intensity over time. The second detector 268 generates a decreasing intensity output signal $1_2$. The interface control circuit 270 converts this signal to the trailing edge 276 of the square pulse 272 shown in Fig. 28B. This event marks the ending time ($T_2$) of the pulse 272.

As Figs. 23A and B show, the interface control circuit 270 measures, for each successive pulse 272A and 272B, the time period between the leading pulse edge 274 ($T_1$ in Fig. 23) and the trailing pulse edge 276 $T_2$ in Fig. 23). This measurement ($T_2$ minus $T_1$) constitutes the length of the pulse (in seconds).

The interface control circuit 270 also preferably measures the time period between two successive pulses (shown as 272A and 272B in Fig. 23C). This period of time is measured between the leading edge 274 of the first pulse 272A ($T_1$ in Fig. 23C) and the leading edge 274 of the next successive pulse 272B ($T_3$ in Fig. 23C) . This measurement constitutes the period of the adjacent pulses (in seconds).

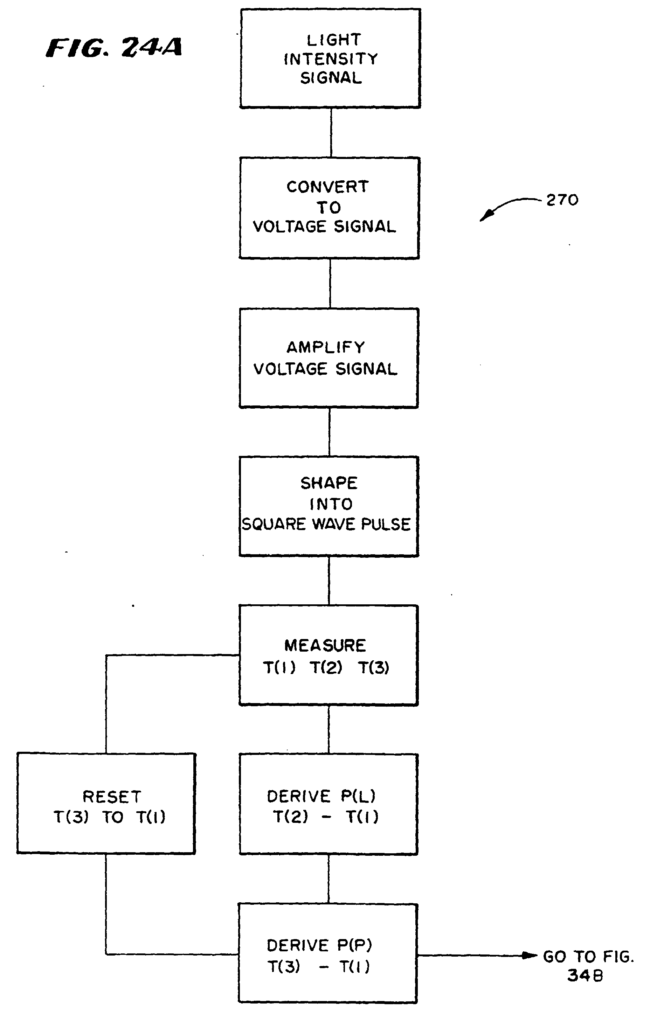

After this measurement has been made, the interface control circuit 270 then resets $T_3$ to $T_1$ for the next pulse measurement cycle (see Fig. 24A).

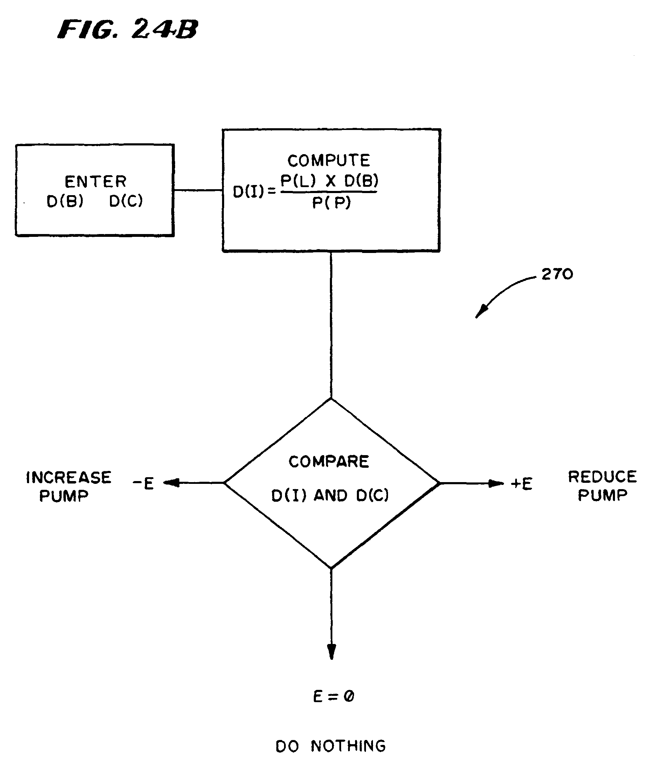

As Fig. 24B shows, the interface control circuit 270 derives the physical dimensions of the red cell band of the interface from these time pulse measurements, based upon the following relationship:

$$\frac{P_L}{P_P} - \frac{D_I}{D_B}$$

where:

$P_L$ is the measured length of the pulse ($T_2$ minus $T_1$) (in seconds);

$P_P$ is the measured period of the pulse ($T_3$ minus is the measured $T_1$) (also in seconds) ;

$D_1$ is the length of the red cell band of the interface (in inches) to be derived; and

$D_B$ is the circumference of the bowl assembly 80 (in inches).

If the rate of rotation of the bowl assembly 80 remains constant during the period of pulse measurements, the reciprocal of the frequency of rotation in seconds ($1/F_{rot}$ in Hz)) can be substituted for $P_P$

Based upon the above relationship, $D_1$ can be derived as follows:

$$D_I = \frac{P_L \times D_B}{P_P}$$

As Fig. 24B shows, the interface control circuit 270 compares the derived physical measurement of the interface $D_1$ with a control value ($D_C$) to generate an error signal (E).

The interface control value $D_C$ can comprise a preselected fixed absolute value (in inches) that the user inputs. Alternatively, the interface control value $D_C$ can be expressed as a percentage based upon the length of the interface ramp 130 (i.e., red cells should occupy no more than 30% of the interface ramp 130).

With reference now also to Fig. 15A, if the error signal (E) is positive, indicating that the red cell band of the interface is too large, the interface control circuit 270 generates a signal to reduce the pumping rate of the plasma pumping station 186/210 (see Fig. 24B). This pushes the RBC region away from the PRP collection port 72 back toward the desired control position (Fig. 15B), where the error signal (E) is zero.

With reference to Fig. 15C, if the error signal (E) is negative, indicating that the red cell band of the interface is too small, the interface control circuit 270 generates a signal to increase the pumping rate of the plasma pumping station 186/210 (see Fig. 24B). This pushes the RBC region toward the PRP collection port 72 back toward the desired control position (Fig. 15B), where the error signal (E) is again zero.

The optical data link 278 described above is representative of a broader class of systems for transmitting a control signal between a rotating element and a stationary element without mechanical contact between the two elements.

Like the illustrated optical data link 278, such a system employs sensor means on either the rotating or stationary element. The sensor means senses an operating condition that is subject to change. The sensor means generates a first output signal that varies according to changes in the sensed operating condition.

Like the illustrated optical data link 278, such a system includes an energy emitter on the one element that carries the sensor means. The emitter emits energy to the other element without mechanical contact with the other element. The emitter modulates the emitted energy according to variations occurring in the intensity of the first output signal. Alternatively, the sensor means itself can constitute an emitter of modulated energy.

The emitted energy used by the data link 278 is light. However, sound energy or other types of electromagnetic energy could be used as well.

Like the illustrated data link 278, the system includes a detector on the other element for receiving the modulated energy emitted by the emitter. The detector demodulates the detected energy to generate a second output signal that, like the first output signal, varies according to the changes in the sensed operating condition.

Such a "connectionless" system for transmitting data between moving and stationary elements would be applicable for use for all sorts of real time control functions, not just interface control.

**Claims**

1. A chamber for use in a rotating field to separate blood components comprising:

a separation channel (84) having a low-G side wall (64) radially spaced from the rotational axis, a high-G side wall (66) radially spaced from the rotational axis farther than the low-G side wall, and end walls that are spaced apart circumferentially about the rotational axis, an inlet port (68) near the first end wall for introducing blood into the channel for flow circumferentially about the rotational axis from the first end wall toward the second end wall for separation into two blood components, and a a first outlet port (70) juxtaposed next to the inlet port (68) near the first end wall for conveying a first separated blood component from the channel, a second outlet port (72) juxtaposed next to the inlet port (68) for conveying the second separated blood component from the channel, and

collection means within the channel including stepped up barrier means (128) near the second end wall to block passage of the second component while directing said first separated blood component to a collection region near the second end wall, including means (116) defining within the channel an enclosed interior collection passage (126) that leads from the collection region and directs the collected, separated component to the first outlet port (70) for transport from the chamber.

2. A chamber for use in a rotating field to separate blood components comprising:

a body defining a separation zone that is divided into contiguous first and second separation channels (84,86), each separation channel having a low-G side wall (64) radially spaced from the rotational axis, a high-G side wall (66) radially spaced from the rotational axis farther than the low-G side wall, and end walls that are spaced apart circumferentially about the rotation axis, the first end wall of the first channel (84) adjoining the first end wall of the second channel (86),

each of the first and second channels (84,86) including an inlet port (68,138) near its associated first end wall for introducing blood or a blood component respectively into the channel for flow circumferentially about the rotational axis from the first end wall toward the second end wall for separation, the inlet ports for the first and second channels thereby being mutually juxtaposed on the chamber body,

each channel including an outlet port (70,136) juxtaposed its associated inlet port for conveying a separated blood constituent from the associated channel, the inlet and outlet ports thereby being mutually io juxtaposed on the chamber body, and the outlet port (70) of the first channel being connected with the inlet port (138) of the second channel (86),

each channel including collection means within the channel the collection means of the first channel (84) including means (128) for directing a separated blood component to a collection region near the second end wall, the collection means of each channel (84,86) including means (116,142) defining within the channel an enclosed interior collection passage (126,146) that leads from the associated collection region and directs the collected, separated component to the outlet port (70,136) of the associated channel.

3. A chamber according to Claim 1 or 2, wherein the axial height of the or each collection passage (126) decreases from the associated collection region to the associated outlet port (70,136).

4. A chamber according to Claim 1, 2 or 3, wherein at least a portion of the low-G side wall (64) of the or each channel (84,86) tapers toward the high-G side wall (66) from the first end wall toward the second end wall.

5. A chamber according to Claim 2, wherein the collection means of the or the first chamber (84) includes barrier means (128) near the second end wall for creating a restricted inlet (129) between the collection region and the associated collection passage (126).

6. A chamber as in any preceding claim, for use in a rotating field to separate whole blood into red blood cells and plasma constituent by introducing whole blood into the or the first channel (84) for flow circumferentially about the rotational axis from the first end wall toward the second end wall for separation of red blood cells toward the high-G side wall (66) and plasma constituent toward the low-G side wall (64), the chamber including

a second outlet port (72) juxtaposed next to the inlet port (68) which is near the first end wall of the or the first channel (84);

second collection means within the channel for directing separated plasma constituent along the low-G side wall (64) to a second collection region near (124) the first end wall, including means (110) for defining a plasma collection passage that leads from the second collection region (124) and directs collected plasma constituent to the second outlet port (72) for transport from the or the first channel (84), and wherein, in use, the first collection means of the or the first channel directs separated red blood cells along the high-G side wall (66) to the first collection region and said interior collection passage (126) directs collected red blood cells to the first outlet port (70) for transport from the or the first channel (84).

7. A chamber according to Claim 6 wherein, in use, when whole blood is introduced into a first region of the or the first channel (84) next to the first end wall, separation of red blood cells is initiated within the first region toward the high-G side wall (66) and a flow of plasma is created that moves within the first region radially toward the low-G side wall (64) and elutes platelets into suspension, and wherein the second collection region (124) lies in the path of the radial flow of plasma and eluted platelets in the first region.

**8.** A chamber according to Claim 6 or 7, wherein the second collection means includes first barrier means (114) near the first end wall for creating a restricted inlet between the second collection region (124) and the plasma collection passage along the low-G side wall (64) of the or the first channel (84).

**9.** A chamber according to Claim 8 wherein the barrier means (114) orients the interface between the red blood cells and the plasma constituent for viewing through one of the side walls (64,66) of the or the first channel (84).

**10.** A chamber according to any preceding claim, wherein the end of the interior collection passage (126) of the or the first collection means that communicates with the collection region presents a larger cross section than the end that communicates with the or the first outlet port (70,136) of the associated channel.

**11.** A chamber according to any preceding claim wherein the interior collection passage (126) of the or the first collection means extends generally circumferentially about the rotational axis.

**12.** A chamber according to any preceding claim including a whole blood inlet passage (122) that leads from the inlet port (68) which is near the first end wall of the or the first channel (84), and directs whole blood into the or the first channel (84).

**13.** A chamber according to Claim 12 wherein the whole blood inlet passage (122) includes a portion that extends generally parallel to the rotational axis and a portion that directs whole blood into circumferential flow within the or the first channel (84).

**14.** The chamber of any preceding claim comprising facing sheets of flexible plastic material sealed about their peripheral edges to enclose the separation channel or channels (84,86), the channels (84,86) each having top and bottom walls spaced apart axially along the rotational axis, with the associated inlet port (68,138) extending through the top wall near the associated first end wall, the associated outlet port or ports (70,72,136) extending through the top wall juxtaposed next to the associated inlet port (68,138), a first interior seal area (110) defining a or the plasma collection passage (124) aligned in flow relationship with a or the second outlet port (72) juxtaposed next to the inlet port (68) which is near the first end wall of the or the first channel (84), for directing collected plasma constituent to the second outlet port (72) for transport from the or the first channel (84), and a second interior seal area (116) defining said interior collection passage of the or the first channel, the interior collection passage (126) being aligned in flow relationship with the first outlet port (70) of the or the first collection means for directing collected red blood cells near the second end wall to the first outlet port (70) for transport from the or the first channel (84).

**Patentansprüche**

**1.** Kammer zur Verwendung in einem Drehfeld, um Blutkomponenten zu trennen, die folgendes aufweist:

einen Trennkanal (84), der eine Nieder-G-Seitenwand (64), die von der Drehachse radial beabstandet ist, eine Hoch-G-Seitenwand (66), die von der Drehachse weiter als die Nieder-G-Seitenwand radial beabstandet ist, und Endwände hat, die umfangsmägig um die Drehachse herum beabstandet sind,
eine Einlaßöffnung (68) nahe der ersten Endwand zum Einleiten von Blut in den Kanal, so daß das Blut zur Auftrennung in zwei Blutkomponenten von der ersten Endwand in Umfangsrichtung um die Drehachse zu der zweiten Endwand fließt, und
eine erste Auslaßöffnung (70), die neben der Einlaßöffnung (68) nahe der ersten Endwand angeordnet ist, um eine erste getrennte Blutkomponente aus dem Kanal abzutransportieren, und
eine zweite Auslagöffnung (72) die neben der Einlaßöffnung (68)' angeordnet ist, um die zweite getrennte Blutkomponente aus dem Kanal abzutransportieren, und
eine Sammeleinrichtung innerhalb des Kanals mit einer aufgestuften Sperreinrichtung (128) nahe der zweiten Endwand um den Durchlaß der zweiten Komponente zu blockieren, während die erste Blutkomponente zu einer Sammelzone nahe der zweiten Endwand geleitet wird, wobei die Sammeleinrichtung eine Einrichtung (116) aufweist, die innerhalb des Kanals einen umschlossenen inneren Sammelkanal (126) definiert, der von dem Sammelbereich wegführt und die gesammelte getrennte Komponente zu der ersten Auslaßöffnung (70) für den Transport aus der Kammer leitet.

**2.** Kammer zur Verwendung in einem Drehfeld, um Blutkomponenten zu trennen, die folgendes aufweist:

einen Körper, der eine Trennzone definiert, die in aneinandergrenzende erste und zweite Trennkanäle (84, 86) unterteilt ist, wobei jeder Trennkanal eine Nieder-G-Seitenwand (64), die von der Drehachse radial beabstandet ist, eine Hoch-G-Seitenwand (66), die von der Drehachse weiter als die Nieder-G-Seitenwand radial beabstandet ist, und Endwände hat, die umfangsmäßig um die Drehachse herum beabstandet sind, wobei die erste Endwand des ersten Kanals (84) an die erste Endwand des zweiten Kanals (86) angrenzt,

wobei jeder der ersten und zweiten Kanäle eine Einlaßöffnung (68, 138) nahe seiner zugehörigen ersten Endwand aufweist, um Blut oder eine Blutkomponente in den Kanal einzuleiten, so daß das Blut oder die Blutkomponente von der ersten Endwand in Umfangsrichtung um die Drehachse zu der zweiten Endwand strömt, um getrennt zu werden, wobei die Einlaßöffnungen für den ersten und den zweiten Kanal dadurch beiderseitig aneinandergrenzend an dem Kammerkörper angeordnet sind,

wobei jeder Kanal eine Auslaßöffnung (70, 136) angrenzend an seine zugehörige Einlaßöffnung aufweist, um einen getrennten Blutbestandteil aus dem zugehörigen Kanal abzutransportieren, wobei die Einlaß- und Auslaßöffnungen dadurch beiderseitig aneinandergrenzend an dem Kammerkörper angeordnet sind und wobei die Auslaßöffnung (70) des ersten Kanals mit der Einlaßöffnung (138) des zweiten Kanals (86) verbunden ist,

wobei jeder Kanal eine Sammeleinrichtung innerhalb des Kanals aufweist, wobei die Sammeleinrichtung des ersten Kanals (84) Mittel (128) aufweist, um eine getrennte Blutkomponente zu einer Sammelzone nahe der zweiten Endwand zu leiten, wobei die Sammeleinrichtung von jedem Kanal (84, 86) Mittel (116, 142) aufweist, die innerhalb des Kanals einen umschlossenen inneren Sammeldurchlaß (126, 146) definieren, der von der zugehörigen Sammelzone wegführt und die gesammelte getrennte Komponente zu der Auslaßöffnung (70, 136) des zugehörigen Kanals leitet.

3. Kammer nach Anspruch 1 oder 2, wobei die axiale Höhe des oder jedes Sammelkanals (126) von der zugehörigen Sammelzone zu der zugehörigen Auslaßöffnung (70, 136) hin abnimmt.

4. Kammer nach Anspruch 1, 2 oder 3, wobei sich wenigstens ein Bereich der Nieder-G-Seitenwand (64) des Kanals oder jedes Kanals (84, 86) in Richtung zu der Hoch-G-Seitenwand (66) von der ersten Endwand in Richtung der zweiten Endwand verjüngt.

5. Kammer nach Anspruch 2, wobei die Sammeleinrichtung der Kammer oder der ersten Kammer (84) eine Sperreinrichtung (128) nahe der zweiten Endwand aufweist, um einen begrenzten Zulauf (129) zwischen der Sammelzone und dem zugehörigen Sammeldurchlaß (126) zu erzeugen.

6. Kammer nach einem der vorhergehenden Ansprüche zur Verwendung in einem Drehfeld, um Vollblut in rote Blutzellen und Plasmabestandteil durch Einleiten von Vollblut in den Kanal oder den ersten Kanal (84) zu trennen, so daß es von der ersten Endwand in Umfangsrichtung um die Drehachse zu der zweiten Endwand strömt, um rote Blutzellen in Richtung zu der Hoch-G-Seitenwand (66) und Plasmabestandteil in Richtung zu der Nieder-G-Seitenwand (64) zu trennen, wobei die Kammer folgendes aufweist:

eine zweite Auslaßöffnung (72), die angrenzend an die Einlaßöffnung (68) angeordnet ist, die nahe der ersten Endwand des Kanals oder des ersten Kanals (84) liegt;

eine zweite Sammeleinrichtung innerhalb des Kanals, um getrennten Plasmabestandteil entlang der Nieder-G-Seitenwand (64) zu einer zweiten Sammelzone nahe (124) der ersten Endwand zu leiten, mit Mitteln (110), die einen Plasmasammeldurchlaß definieren, der von der zweiten Sammelzone (124) wegführt und gesammelten Plasmabestandteil zu der zweiten Auslaßöffnung (72) leitet, um aus dem Kanal oder dem ersten Kanal (84) abtransportiert zu werden, und wobei im Gebrauch die erste Sammeleinrichtung des Kanals oder des ersten Kanals getrennte rote Blutzellen entlang der Hoch-G-Seitenwand (66) zu der ersten Sammelzone leitet und der innere Sammeldurchlaß (126) gesammelte rote Blutzellen zu der ersten Auslaßöffnung (70) zum Transport aus dem Kanal oder dem ersten Kanal (84) leitet.

7. Kammer nach Anspruch 6, wobei im Gebrauch, wenn Vollblut in eine erste Zone des Kanals oder des ersten Kanals (84) nahe der ersten Endwand eingeleitet wird, die Trennung von roten Blutzellen innerhalb der ersten Zone in Richtung zu der Hoch-G-Seitenwand (66) initiiert wird und ein Fluß von Plasma erzeugt wird, der sich innerhalb der ersten Zone radial zu der Nieder-G-Seitenwand (64) hin bewegt und Plättchen in Suspension eluiert, und

wobei die zweite Sammelzone (124) in der Bahn der radialen Plasmaströmung und der eluierten Plättchen in der ersten Zone liegt.

**8.** Kammer nach Anspruch 6 oder 7, wobei die zweite Sammeleinrichtung eine erste Sperreinrichtung (114) nahe der ersten Endwand aufweist, um einen begrenzten Zulauf zwischen der zweiten Sammelzone (124) und dem Plasmasammeldurchlaß entlang der Nieder-G-Seitenwand (64) des Kanals oder des ersten Kanals (84) zu erzeugen.

**9.** Kammer nach Anspruch 8, wobei die Sperreinrichtung (114) die Grenzfläche zwischen den roten Blutzellen und dem Plasmabestandteil orientiert, so daß sie durch eine der Seitenwände (64, 66) des Kanals oder des ersten Kanals (84) sichtbar ist.

**10.** Kammer nach einem der vorhergehenden Ansprüche, wobei das Ende des inneren Sammeldurchlasses (126) der Sammeleinrichtung oder der ersten Sammeleinrichtung, der mit der Sammelzone kommuniziert, einen größeren Querschnitt als das Ende aufweist, das mit der Auslaßöffnung oder der ersten Auslaßöffnung (70, 136) des zugehörigen Kanals kommuniziert.

**11.** Kammer nach einem der vorhergehenden Ansprüche, wobei der innere Sammeldurchlaß (126) der Sammeleinrichtung oder der ersten Sammeleinrichtung sich allgemein umfangsmäßig um die Drehachse erstreckt.

**12.** Kammer nach einem der vorhergehenden Ansprüche, der einen Vollbluteinlaßdurchlaß (122) aufweist, der von der Einlaßöffnung (68), die nahe der ersten Endwand des Kanals oder des ersten Kanals (84) angeordnet ist, wegführt und Vollblut in den Kanal oder den ersten Kanal (84) leitet.

**13.** Kammer nach Anspruch 12, wobei der Vollbluteinlaßdurchlaß (122) einen Bereich, der sich allgemein parallel zu der Drehachse erstreckt, und einen Bereich aufweist, der Vollblut in eine Umfangsströmung innerhalb des Kanals oder des ersten Kanals (84) leitet.

**14.** Kammer nach einem der vorhergehenden Ansprüche, die folgendes aufweist: einander zugewandte Flächenkörper aus flexiblem Kunststoff, die um ihre Augenränder herum abgedichtet sind, um den Trennkanal oder die -kanäle (84, 86) zu umschließen, wobei die Kanäle (84, 86) jeweils obere und untere Wände haben, die in Axialrichtung entlang der Drehachse voneinander beabstandet sind, wobei die zugehörige Einlaßöffnung (68, 138) durch die obere Wand nahe der zugehörigen ersten Endwand verläuft, die zugehörige Auslaßöffnung oder -öffnungen (70, 72, 136) durch die obere Wand neben der zugehörigen Einlaßöffnung (68, 138) verläuft oder verlaufen, ein erster innerer Abdichtbereich (110) einen oder den Plasmasammeldurchlaß (124) definiert, der in Strömungsbeziehung mit einer oder der zweiten Auslaßöffnung (72) ausgerichtet ist, die als nächstes neben der Einlaßöffnung (68) angeordnet ist, die nahe der ersten Endwand des Kanals oder des ersten Kanals (84) ist, um gesammelten Plasmabestandteil zu der zweiten Auslaßöffnung (72) zum Transport aus dem Kanal oder dem ersten Kanal (84) zu leiten, und ein zweiter innerer Abdichtbereich (116) den inneren Sammeldurchlaß des oder des ersten Kanals definiert, wobei der innere Sammeldurchlaß (126)in Strömungsbeziehung mit der ersten Auslaßöffnung (70) der Sammeleinrichtung oder der ersten Sammeleinrichtung ausgerichtet ist, um gesammelte rote Blutzellen nahe der zweiten Endwand zu der ersten Auslaßöffnung (70) zum Transport aus dem Kanal oder dem ersten Kanal (84) zu leiten.

**Revendications**

**1.** Chambre, destinée à être utilisée dans un champ rotatif pour séparer des constituants du sang, comprenant :

un conduit de séparation (84) comportant une paroi latérale de faible G (64), espacée radialement de l'axe de rotation, une paroi latérale de G élevé (66), espacée radialement de l'axe de rotation d'une distance plus grande que la paroi latérale de faible G, et des parois d'extrémité qui sont espacées circonférentiellement autour de l'axe de rotation, un orifice d'entrée (68) disposé au voisinage de la première paroi d'extrémité et servant à introduire du sang dans le conduit de façon qu'il s'écoule circonférentiellement autour de l'axe de rotation, de la première paroi d'extrémité vers la seconde paroi d'extrémité, en vue de sa séparation en deux constituants du sang, un premier orifice de sortie (70) juxtaposé directement à l'orifice d'entrée (68) au voisinage de la première paroi d'extrémité et servant à évacuer do conduit un premier constituant du sang séparé, un second orifice du sortie (72) juxtaposé directement à l'orifice d'entrée (68) pour évacuer du conduit le second constituant du sang séparé et, des moyens collecteurs qui sont disposés dans le conduit et comprennent des moyens formant barrière en surélévation (128) au voisinage de la seconde paroi d'extrémité afin de bloquer le passage du second constituant tout en dirigeant le premier constituant du sang séparé jusqu'à une zone collectrice située au voisinage de la seconde paroi d'extrémité et qui comprennent des moyens (116) délimitant, dans le conduit, un passage collecteur intérieur entouré (126) qui s'éloigne de la zone collectrice et dirige le constituant séparé recueilli jusqu'au premier orifice de sortie (70) en vue de son transport hors de la chambre.

2. Chambre, destinée à être utilisée dans un champ rotatif pour séparer des constituants du sang, comprenant :

un corps délimitant une zone de séparation qui est divisée en un premier et un second conduits dc séparation (84, 86) contigus, chaque conduit de séparation comportant une paroi latérale de faible G (64), espacée radialement de l'axe de rotation, une paroi latérale de G élevé (66), espacée radialement de l'axe de rotation d'une distance plus grande que la paroi latérale de faible G, et des parois d'extrémité qui sont espacées circonférentiellement autour de l'axe de rotation, la première paroi d'extrémité du premier conduit (84) étant attenante à la première paroi d'extrémité du second conduit (86),
chacun des premier et second conduits (84, 86) comportant un orifice d'entrée (68, 138), disposé au voisinage de la première paroi d'extrémité qui lui est associée, et servant à introduire respectivement du sang ou un constituant du sang dans le conduit de façon qu'il s'écoule circonférentiellement autour de l'axe de rotation, de la première paroi d'extrémité vers la seconde paroi d'extrémité, en vue de sa séparation, les orifices d'entrée des premier et second conduits étant ainsi juxtaposés l'un à l'autre sur le corps de chambre,
Chaque conduit comportant un orifice de sortie (70, 136) juxtaposé directement à l'orifice d'entrée (68) qui lui est associé, et servant à évacuer du conduit qui lui est associé un constituant du sang séparé, les orifices d'entrée et de sortie étant ainsi juxtaposés les uns aux autres sur le corps de chambre et l'orifice de sortie (70) du premier conduit étant relit à l'orifice d'entrée (138) du second conduit (86),
chaque conduit comportant des moyens collecteurs qui sont disposés dans le conduit, les moyens collecteurs du premier conduit (84) comprenant des moyens (128) servant à diriger un constituant du sang séparé jusqu'à une zone collectrice située au voisinage de la seconde paroi d'extrémité, les moyens collecteurs de chaque conduit (84,86) comportant des moyens (116, 142) délimitant dans le conduit, un passage collecteur intérieur entouré (126, 146) qui s'éloigne de la zone collectrice associée et dirige le constituant séparé recueilli jusqu'à l'orifice de sortie (70, 136) du conduit associé.

3. Chambre selon la revendication 1 ou 2, dans laquelle la hauteur axiale du passage collecteur ou de chaque passage collecteur (126) diminue de la zone collectrice associée jusqu'à l'orifice de sortie associé (70, 136).

4. Chambre selon la revendication 1, 2 ou 3, dans laquelle au moins une partie de la paroi latérale de faible G (64) du conduit ou de chaque conduit (84, 86) présente une distance vis-à-vis de la paroi latérale G élevé (66) qui diminue de la première paroi d'extrémité vers la seconde paroi d'extrémité.

5. Chambre selon la revendication 2, dans laquelle les moyens collecteurs du conduit ou du premier conduit (84) comprennent des moyens formant barrière (128) située au voisinage de la seconde paroi d'extrémité et servant à créer une entrée étranglée (129) entre la zone collectrice et le passage collecteur (126) associé.

6. Chambre selon l'une quelconque des revendications précédentes, destinée à être utilisée dans un champ rotatif pour séparer du sang complet en globules rouges du sang et constituant formé de plasma, en introduisant du sang complet dans le conduit ou le premier conduit (84) de façon qu'il s'écoule circonférentiellement autour de l'axe de rotation, de la première paroi d'extrémité vers la seconde paroi d'extrémité, en vue d'une séparation des globules rouges du sang vers la paroi latérale de G élevé (66) et du constituant formé de plasma vers la paroi latérale de faible G (64), la chambre comportant :

un second orifice de sortie (72) juxtaposé directement à l'orifice d'entrée (68) qui est situé au voisinage de la première paroi d'extrémité du conduit ou du premier conduit (84),
des seconds moyens collecteurs qui sont situés dans le conduit et servent à diriger le constituant formé de plasma séparé, le long de la paroi latérale à faible G (64), jusqu'à une seconde zone collectrice située au voisinage (124) de la première paroi d'extrémité, et qui comprennent des moyens (110) servant à délimiter un passage collecteur de plasma qui s'éloigne de la seconde zone collectrice (124) et dirige le constituant formé de plasma recueilli jusqu'au second orifice de sortie (72), en vue de son transport hors du conduit ou du premier conduit (84), tandis qu'en cours d'utilisation, les premiers moyens collecteurs du conduit ou du premier conduit dirigent les globules rouges du sang séparés, le long de la paroi latérale de G élevé (66), jusqu'à la première zone collectrice et le passage collecteur intérieur (126) dirige les globules rouges du sang recueillis jusqu'au premier orifice de sortie (70), en vue de leur transport hors du conduit ou du premier conduit (84).

7. Chambre selon la revendication 6, dans laquelle, en cours d'utilisation, lorsque du sang complet est introduit dans une première zone du conduit ou du premier conduit (84) située au voisinage de la première paroi d'extrémité, une séparation des globules rouges du sang est amorcée dans la première zone vers la paroi latérale de G élevé (66), tandis qu'il se crée un écoulement de plasma qui se déplace dans la première zone radialement en direction de la paroi latérale de faible G et sépare les plaquettes en suspension par élution et dans laquelle la seconde zone collectrice (124) est située sur le trajet de l'écoulement radial de plasma et de plaquettes séparées par élution, dans la première zone.

8. Chambre selon la revendication 6 ou 7, dans laquelle les seconds moyens collecteurs comprennent des premiers moyens formant barrière (114) situés au voisinage de la première paroi d'extrémité et servant à créer une entrée étranglée entre la seconde zone collectrice (124) et la passage collecteur de plasma, le long de la paroi latérale de faible G (64) du conduit ou du premier conduit (84).

9. Chambre selon la revendication 8, dans laquelle les moyens formant barrière (114) orientent l'interface située entre les globules rouges du sang et le constituant formé de plasma an vue d'une observation à travers l'une des parois latérales (64, 66) du conduit ou du premier conduit (84).

10. Chambre selon l'une quelconque des revendications précédentes, dans laquelle l'extrémité du passage collecteur intérieur (126) des moyens collecteurs ou des premiers moyens collecteurs qui communique avec la zone collectrice présente une section transversale plus grande que l'extrémité qui communique avec l'orifice de sortie ou le premier orifice de sortie (70, 136) du conduit associé.

11. Chambre selon l'une quelconque des revendications précédentes, dans laquelle le passage collecteur intérieur (126) des moyens collecteurs ou des premiers moyens collecteurs s'étend dans l'ensemble circonférentiellement autour de l'axe de rotation.

12. Chambre selon l'une quelconque des revendications précédentes, comprenant un passage d'entrée de sang complet (122) qui s'éloigne de l'orifice d'entrée (68) qui est situé au voisinage de la première paroi d'extrémité du conduit ou du premier conduit (84), et dirige le sang complet dans le conduit ou le premier conduit (84).

13. Chambre selon la revendication 12, dans laquelle le passage d'entrée de sang complet (122) comporte une partie qui s'étend dans l'ensemble parallèlement à l'axe de rotation et une partie qui dirige le sang complet suivant un écoulement circonférentiel dans le conduit ou le premier conduit (84).

14. Chambre selon l'uns quelconque des revendications précédentes, comprenant des feuilles de matière plastique flexible qui se font face et sont scellées tout autour de leurs bords périphériques de façon à entourer le conduit ou les conduits de séparation (84, 86), les conduits (84, 86) comportant chacun des parois supérieure et inférieure espacées axialement le long de l'axe de rotation, l'orifice d'entrée (68, 138) associé traversant la paroi supérieure au voisinage de la première paroi d'extrémité associée, le ou les orifices de sortie (70, 72, 136) associés traversant la paroi supérieure en étant juxtaposés directement à l'orifice d'entrée (68, 138) associé, une première zone de scellement intérieur (110) définissant un passage ou le passage collecteur de plasma (124) aligné suivant une disposition relative d'écoulement avec un second ou le second orifice de sortie (72) juxtaposé directement à l'orifice d'entrée (68) qui est situé au voisinage de la première paroi d'extrémité du conduit ou du premier conduit (84), servant à diriger le constituant formé de plasma recueilli vers le second orifice de sortie (72) en vue de son transport hors du conduit ou du premier conduit (84), et une seconde zone de scellement intérieur (116) définissant le passage collecteur intérieur du conduit ou du premier conduit, le passage collecteur intérieur (126) étant aligné suivant une disposition relative d'écoulement avec le premier orifice de sortie (70) des moyens collecteurs ou des premiers moyens collecteurs en vue de diriger les globules rouges du sang recueillis, au voisinage de la seconde paroi d'extrémité, jusqu'au premier orifice de sortie (70) afin de les transporter hors du conduit ou du premier conduit (84).

## FIG. 1

## FIG. 2

FIG. 3

o = RBC

$\bigcirc$ = WHITE BLOOD CELLS

• = PLATELETS

*FIG. 4*
PRIOR ART

58A

RBC ←

70

68

WB

72

→ PRP

*FIG. 5*
PRIOR ART

58B

70

RBC ←

WB →

68

72

→ PRP

FIG. 6

FIG. 7

FIG. 8

FIG. 9

EP 0 619 752 B2

**FIG. 10**

FIG. 11

FIG. 12

*FIG. 13*

*FIG. 14*

## FIG. 15A

## FIG. 15B

## FIG. 15C

*FIG. 16*

EP 0 619 752 B2

**FIG. 17**

# FIG. 18

FIG. 19

EP 0 619 752 B2

FIG. 20

FIG. 21A

**FIG. 21B**

**FIG. 22**

MOTOR POWER

236

254

252

260

262

GROUND

266

264

GROUND

244

MOTOR POWER

85

252

240    236    238

246

250

248

130

242    82    80

EP 0 619 752 B2

FIG. 23A

FIG. 23B

FIG. 23C

**FIG. 24A**

```
┌─────────────────┐
│     LIGHT       │
│   INTENSITY     │
│     SIGNAL      │
└─────────────────┘
         │
         │                              ╱ 270
┌─────────────────┐
│     CONVERT     │
│       TO        │
│ VOLTAGE SIGNAL  │
└─────────────────┘
         │
┌─────────────────┐
│     AMPLIFY     │
│  VOLTAGE SIGNAL │
└─────────────────┘
         │
┌─────────────────┐
│      SHAPE      │
│      INTO       │
│SQUARE WAVE PULSE│
└─────────────────┘
         │
┌─────────────────┐
│    MEASURE      │
│ T(1) T(2) T(3)  │
└─────────────────┘
         │
┌───────────┐   ┌─────────────────┐
│   RESET   │   │   DERIVE P(L)    │
│ T(3) TO T(1)│ │   T(2) − T(1)   │
└───────────┘   └─────────────────┘
         │              │
         │     ┌─────────────────┐
         └─────│   DERIVE P(P)    │──► GO TO FIG.
               │   T(3) − T(1)    │        34B
               └─────────────────┘
```

# FIG. 24B

```
┌─────────────┐        ┌──────────────────────┐
│   ENTER     │        │      COMPUTE         │
│             │────────│         P(L) X D(B)  │
│  D(B)  D(C) │        │  D(I) = ───────────  │
│             │        │            P(P)      │
└─────────────┘        └──────────────────────┘
```

— 270

```
                    ◇
INCREASE   -E  ◄─── COMPARE ───►  +E   REDUCE
PUMP                D(I) AND D(C)        PUMP
                    ◇
                    │
                    ▼
```

E = 0

DO NOTHING

## FIG. 25A

## FIG. 25B